(19) Europäisches Patentamt
European Patent Office
Office européen des brevets

(11) **EP 3 498 198 B1**

(12) **EUROPÄISCHE PATENTSCHRIFT**

(45) Veröffentlichungstag und Bekanntmachung des Hinweises auf die Patenterteilung:
**11.03.2020   Patentblatt 2020/11**

(51) Int Cl.:
*A61B 17/29* (2006.01)       *A61B 17/32* (2006.01)
*A61B 17/00* (2006.01)       *A61B 17/3203* (2006.01)
*A61B 34/30* (2016.01)

(21) Anmeldenummer: **18208274.3**

(22) Anmeldetag: **26.11.2018**

(54) **MINIMALINVASIVES MEDIZINISCHES INSTRUMENT**

MINIMALLY INVASIVE MEDICAL INSTRUMENT

INSTRUMENT MÉDICAL À INVASION MINIMALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.12.2017   DE 102017222865**

(43) Veröffentlichungstag der Anmeldung:
**19.06.2019   Patentblatt 2019/25**

(73) Patentinhaber: **Richard Wolf GmbH**
**75438 Knittlingen (DE)**

(72) Erfinder: **Teichtmann, Elmar**
**75015 Bretten (DE)**

(74) Vertreter: **Patentanwälte Vollmann Hemmer Lindfeld**
**Partnerschaft mbB**
**Wallstraße 33a**
**23560 Lübeck (DE)**

(56) Entgegenhaltungen:
**EP-A2- 1 842 500       US-A1- 2007 106 317**
**US-A1- 2017 095 264       US-B2- 7 963 963**

**Beschreibung**

Die vorliegende Offenbarung betrifft ein längliches minimalinvasives medizinisches Instrument, wie etwa ein endoskopisches Instrument, an dessen distalem Ende sich ein beweglicher und/oder betätigbarer Instrumentenkopf befindet. Insbesondere betrifft die Offenbarung Instrumente, bei denen der Instrumentenkopf fluidisch antreibbar und/oder steuerbar ist.

Minimalinvasive medizinische Instrumente mit fluidisch antreibbarem und/oder steuerbarem Instrumentenkopf am distalen Ende sind bereits bekannt. Beispielsweise beschreibt die DE 43 19 345 C1 ein chirurgisches Instrument, mit dem über eine Hydraulikmittelleitung mittels Hydraulikdruck eine Schere am distalen Ende betätigt wird. Ein ähnliches Instrument ist auch aus der US 2006/0235368 A1 bekannt.

Aus der DE 197 42 669 A1 ist ein Instrument bekannt, bei dem ein distalseitiger Fräskopf mit einem auf dem Gerotor-Prinzip basierenden Mikromotor gekoppelt ist, wobei der Mikromotor über einen Antriebskanal fluidisch angetrieben ist.

Die US 7,963,963 B2 beschreibt ein Gefäßklemmgerät mit einem rotierbaren und abwinkelbaren Zangenkopf.

Die US 2007/095264 A1 beschreibt abwinkelbare Gelenke für endoskopische Geräte, wobei die Gelenke fluidbetriebene Druckzylinder aufweisen.

Eine technische Herausforderung bei solchen bekannten Instrumenten mit einem typischen Durchmesser von wenigen Millimetern ist die Ausführung der Hydraulikmittelleitung bzw. des Antriebskanals, wenn der Instrumentenkopf gegenüber der Längsachse des Instruments über ein Gelenk abwinkelbar ist. Dies kann zwar durch eine flexible Schlauchdurchführung durch das Gelenk realisiert werden, allerdings hat dies einige Nachteile. Zum einen benötigt eine flexible Schlauchdurchführung immer einen minimalen Biegeradius, was den Bewegungsspielraum des Gelenks und damit die Anwendungsmöglichkeiten beschränkt. Zum anderen muss die Biegesteifigkeit einer flexiblen Hydraulikmittelleitung beim Abwinkeln überwunden werden, wobei die Biegesteifigkeit vom Hydraulikdruck abhängig ist. Bei einer sehr weichen Hydraulikmittelleitung besteht wiederum das Problem, dass sich der Leitungsquerschnitt mit dem Abwinklungsgrad verändern kann.

Das minimalinvasive medizinische Instrument gemäß der vorliegenden Offenbarung hat demgegenüber den Vorteil, dass der Bewegungsspielraum des Gelenks und damit die Anwendungsmöglichkeiten nicht durch die Fluidleitung beschränkt sind. Außerdem ist das zum Abwinkeln benötigte Drehmoment im Wesentlichen unabhängig vom Druck in der Fluidleitung. Schließlich ist der Querschnitt der Fluidleitung bei jedem Grad der Abwinklung genau definiert.

Gemäß der vorliegenden Offenbarung wird ein minimalinvasives medizinisches Instrument bereitgestellt mit einem sich in einer Längsrichtung erstreckenden Schaft, einem sich an einem distalen Ende des Schafts befindlichen beweglichen und/oder betätigbaren Instrumentenkopf, einem proximalen Schaftabschnitt und einem distalen Schaftabschnitt, wobei der distale Schaftabschnitt gegenüber dem proximalen Schaftabschnitt über ein Gelenk um eine Schwenkachse abwickelbar ist, und zumindest einem vom proximalen Schaftabschnitt in den distalen Schaftabschnitt führenden Fluidkanal. Dabei weist das Gelenk ein erstes Gelenkteil und ein gegenüber dem ersten Gelenkteil um die Schwenkachse verschwenkbares zweites Gelenkteil auf. Zudem weist der zumindest eine Fluidkanal einen ersten Kanalabschnitt im ersten Gelenkteil, einen zweiten Kanalabschnitt im zweiten Gelenkteil und einen sich ringförmig um die Schwenkachse erstreckenden dritten Kanalabschnitt auf. Der dritte Kanalabschnitt weist dabei einen ersten vom ersten Gelenkteil gebildeten Ringwandungsabschnitt und einen zweiten vom zweiten Gelenkteil gebildeten Ringwandungsabschnitt auf.

Unter "ringförmig" sei hier ein vollständiges (360°) oder nur teilweises (<360°) rotationssymmetrisches Erstrecken um die Schwenkachse zu verstehen. Zwar ist ein vollständiges (360°) Erstrecken hier bevorzugt, um den Bewegungsspielraum des Gelenks nicht durch den dritten Kanalabschnitt zu begrenzen, aber der Bewegungsspielraum des Gelenks kann ohnehin aus anderen Gründen beschränkt sein, sodass ein entsprechend nur teilweises (<360°) rotationssymmetrisches Erstrecken des dritten Kanalabschnitts ausreichen kann. Die Querschnittsform des ringförmigen dritten Kanalabschnitts kann beliebig sein, beispielsweise kreisförmig, wodurch sich beispielsweise ein torusförmiger dritter Kanalabschnitt ergibt.

Der Instrumentenkopf kann eine Lampe, eine Kamera, eine Spüldüse, eine Absaugstelle, eine Abrasivstrahldüse, eine Schere, eine Zange, einen Greifer, einen Fräskopf, einen Schleifer und/oder ein ähnliches Werkzeug für einen minimalinvasiven medizinischen Eingriff in einen Körper eines Patienten aufweisen. Proximalseitig kann das Instrument mit einem Handgriff, einem Motorgriff und/oder einem Roboterarm fest verbunden oder koppelbar sein, unmittelbar oder mittelbar über einen Adapter oder eine Kopplung.

Der mindestens eine Fluidkanal kann vorzugsweise dazu ausgestaltet sein, eine Hydraulikflüssigkeit zum fluidischen Antreiben und/oder Steuern des beweglichen und/oder betätigbaren Instrumentenkopfs zu führen. Allerdings kann der Fluidkanal alternativ dazu oder zusätzlich als pneumatischer Kanal Luft oder ein anderes Gas führen. Ein Fluidkanal kann mit einer distalseitigen Absaugstelle fluidverbunden sein, sodass Körperflüssigkeiten, Spülflüssigkeiten, Abrasiv-Schneidmittel und/oder Körpergewebe über den Fluidkanal absaugbar sind. Alternativ oder zusätzlich kann ein durch den Fluidkanal führbares Fluid als Antriebsmittel, als Spülmittel und/oder als Abrasiv-Schneidmittel eingesetzt werden. Als Spül- oder Abrasiv-Schneidmittel wird vorzugsweise ein mit dem Körper des Patienten verträgliches steriles Fluid wie etwa isotonische 0,9%ige Salzlösung eingesetzt. Auch als Antriebsmittel ist ein solches steriles bioverträgliches Fluid bevorzugt, da im Falle einer Leckage kein Schaden für den Patienten entsteht. Ein steriles bioverträgliches Fluid wie etwa isotonische 0,9%ige Salzlösung kann sowohl als Antriebsmittel als auch als Spülmittel dienen, wenn zunächst mit dem Fluid mechanische Arbeit verrichtet wird und das Fluid anschließend über einen Spülauslass abgelassen wird.

Optional kann sich der zumindest eine Fluidkanal zumindest teilweise in Richtung der Schwenkachse erstrecken. Dies vereinfacht es, zwischen dem ersten Ringwandungsabschnitt und dem zweiten Ringwandungsabschnitt mindestens eine zur Schwenkachse ro-tationssymmetrische Dichtlinie vorzusehen. Vorzugs-weise grenzt der erste Ringwandungsabschnitt über zwei zur Schwenkachse koaxiale kreisförmige Dichtlinien an den zweiten Ringwandungsabschnitt, sodass der ringförmige dritte Kanalabschnitt vollständig durch den ersten Ringwandungsabschnitt und den zweiten Ringwandungsabschnitt gebildet ist.

Optional kann das Gelenk einen Achskörper aufweisen, der sich in Richtung der Schwenkachse und durch den ringförmigen Kanalabschnitt erstreckt. Der Achskörper kann dabei ein Achsstift oder -bolzen sein, der sich durch die beiden Gelenkteile erstreckt, oder als männlicher Teil eines der Gelenkteile ausgebildet sein. Als männlicher Teil eines Gelenkteils kann der Achskörper integraler Bestandteil des Gelenkteils sein oder bei der Herstellung bzw. Montage des Instruments mit dem Gelenkteil fest verbunden werden (z.B. durch Verkleben, Verschweißen, Verpressen, Verschrauben oder ein ähnliches Verbindungsverfahren). Das andere Gelenkteil kann dann eine entsprechende weibliche Aufnahme aufweisen, in die der männliche Achskörper des anderen Gelenkteils während der Herstellung bzw. Montage des Instruments passgenau eingeführt werden kann.

Optional kann der erste Kanalabschnitt gegenüber dem zweiten Kanalabschnitt bezüglich der Längsrichtung zumindest streckenweise radial versetzt zueinander verlaufen. Dies kann einem axialen Versatz bezüglich der Schwenkachse entsprechen, sodass dieser Versatz mit einem in Richtung der Schwenkachse verlaufenden Kanalabschnitt geschlossen werden kann.

In einer beispielhaften Ausführungsform können der erste Ringwandungsabschnitt und der zweite Ringwandungsabschnitt in axialer Richtung der Schwenkachse aneinander angrenzen. Der dritte Kanalabschnitt kann dabei bezüglich einer zur Schwenkachse orthogonalen Dichtungsebene spiegelsymmetrisch aufgebaut sein, wobei der erste Ringwandungsabschnitt in Form einer oberen Hälfte und der zweite Ringwandungsabschnitt in Form einer unteren Hälfte zusammen den dritten Kanalabschnitt bilden. Optional kann das erste Gelenkteil und das zweite Gelenkteil jeweils im Wesentlichen planare Kontaktflächen aufweisen, die in einer zur Schwenkachse orthogonalen Dichtungsebene um die Schwenkachse herum dichtend aneinander liegen. Die zwei zur Schwenkachse koaxialen kreisförmigen Dichtlinien zwischen den Ringwandungsabschnitten haben in dieser beispielhaften Ausführungsform unterschiedliche Radien (Innen- und Außenradius des ringförmigen dritten Kanalabschnitts) und liegen beide in der Dichtungsebene.

In dieser beispielhaften Ausführungsform können die Gelenkteile symmetrisch zueinander oder identisch aufgebaut sein, was die Fertigungskosten reduziert. Die Dichtungsebene zwischen den Gelenkteilen kann durch eine vom als Achsstift oder -bolzen ausgebildeten Achskörper ausgeübte Axialkraft in Richtung der Schwenkachse dichtend zusammengehalten werden. Nachteilig bei dieser Ausführungsform ist, dass die Axialkraft bezüglich der Schwenkachse den Reibungswiderstand des Gelenks gegen das Abwinkeln erhöhen kann.

Optional kann das Gelenk ein um die Schwenk-achse verlaufendes ringförmiges Dichtelement aufwei-sen, das zwischen dem ersten Gelenkteil und dem zweiten Gelenkteil dichtend angeordnet ist. In Bezug auf diese beispielhafte Ausführungsform können umlaufende ringförmige Dichtelemente in der Dichtungsebene, die bezüglich der Schwenkachse radialseitig außen und/oder innen vom dritten Kanalabschnitt angeordnet sind, die Dichtigkeit verbessern.

In einer weiteren beispielhaften Ausführungsform können der erste Ringwandungsabschnitt und der zweite Ringwandungsabschnitt in radialer Richtung bezüglich der Schwenkachse aneinander angrenzen. Der dritte Kanalabschnitt kann dabei bezüglich einer zur Schwenkachse koaxialen Dichtungsmantelfläche zweiteilig aus einem inneren ersten Ringwandungsabschnitt und einem äußeren zweiten Ringwandungsabschnitt aufgebaut sein. Optional kann das erste Gelenkteil und das zweite Gelenkteil jeweils im Wesentlichen zylindermantelförmige Kontaktflächen aufweisen, die in einer zur Schwenkachse koaxialen Dichtungsmantelfläche um die Schwenkachse herum dichtend radial aneinander liegen. Die zwei zur Schwenkachse koaxialen kreisförmigen Dichtlinien zwischen den Ringwandungsabschnitten haben in dieser beispielhaften Ausführungsform vorzugsweise den gleichen Radius (mittlerer Radius des ringförmigen dritten Kanalabschnitts oben und unten) und liegen beide vorzugsweise in der Dichtungsmantelfläche. Vorteilhaft gegenüber der zuvor beschriebenen Ausführungsform ist, dass bei dieser Ausführungsform keine Axialkraft in Richtung der Schwenkachse für die Dichtigkeit sorgen muss und somit der Reibungswiderstand des Gelenks geringer ist. Nachteilig ist, dass die Gelenkteile nicht symmetrisch zueinander oder identisch ausgestaltet sind.

Optional kann das Gelenk auch in dieser zweiten beispielhaften Ausführungsform ein um die Schwenkachse verlaufendes ringförmiges Dichtelement aufweisen, das zwischen dem ersten Gelenkteil und dem zweiten Gelenkteil dichtend angeordnet ist. Hierbei können umlaufende ringförmige Dichtelemente in der Dichtungsmantelfläche, die bezüglich der Schwenkachse axial oberhalb und/oder unterhalb vom dritten Kanalabschnitt angeordnet sind, die Dichtigkeit verbessern.

Optional kann das Gelenk einen Achskörper aufweisen, der sich in Richtung der Schwenkachse und durch den ringförmigen Kanalabschnitt erstreckt, wobei der Achskörper einen Teil des ersten Gelenkteils bildet und drehfest mit diesem verbunden ist. Der Achskörper kann integraler Bestandteil des ersten Gelenkteils sein oder während der Herstellung bzw. Montage des Instruments drehfest mit diesem verbunden werden (z.B. durch Verkleben, Verschweißen, Verpressen, Verschrauben oder ein ähnliches Verbindungsverfahren). Der Achskörper kann vorzugsweise als männlicher Teil des ersten Gelenkteils ausgestaltet sein, der während der Herstellung bzw. Montage des Instruments in eine entsprechend passgenaue weibliche Aufnahme im zweiten Gelenkteil in Richtung der Schwenkachse einführbar ist.

Optional kann ein Teil des ersten Kanalabschnitts durch den Achskörper verlaufen. Dabei kann der Außenradius des Achskörpers so groß gewählt werden, dass der erste Kanalabschnitt zumindest teilweise in Richtung der Schwenkachse durch den Achskörper und darin radial nach außen führen kann.

Optional kann der erste Ringwandungsabschnitt durch eine bezüglich der Schwenkachse radiale Außenfläche des Achskörpers gebildet werden. Beispielsweise kann der erste Ringwandungsabschnitt als umlaufende Nut auf der Mantelaußenfläche des Achskörpers ausgebildet sein. Diese Nut kann mit einem Ende des ersten Kanalabschnitts im Inneren des Achskörpers fluidverbunden sein, wobei das andere Ende des ersten Kanalabschnitts im Inneren des Achskörpers an mindestens einer der Stirnseiten des Achskörpers in den übrigen Teil des ersten Gelenkteils führt und in ein proximalseitiges Ende des ersten Gelenkteils mündet. Die radiale Außenfläche des Achskörpers bildet dabei den radial inneren ersten Ringwandungsabschnitt des dritten Kanalabschnitts.

Optional kann der zweite Ringwandungsabschnitt durch eine bezüglich der Schwenkachse radiale Innenfläche des zweiten Gelenkteils gebildet sein. Beispielsweise kann der zweite Ringwandungsabschnitt als umlaufende Nut auf der Mantelinnenfläche einer weiblichen Achskörper-Aufnahme des zweiten Gelenkteils ausgebildet sein. Die radiale Innenfläche des zweiten Gelenkteils bildet dabei den radial äußeren zweiten Ringwandungsabschnitt des dritten Kanalabschnitts. Die den Achskörper umgreifende Aufnahme des zweiten Gelenkteils kann einen inneren zweiten Kanalabschnitt aufweisen, der eine Fluidverbindung zwischen der umlaufenden Nut auf der Mantelinnenfläche der weiblichen Achskörper-Aufnahme und dem distalseitigen Ende des zweiten Gelenkteils bildet.

Die zuvor beschriebenen Ausführungsformen können auch kombiniert werden. Beispielsweise kann die weibliche Achskörper-Aufnahme topfförmig ausgestaltet sein, in die der Achskörper sowohl axial als auch radial genau passt. Der Topfboden kann zusammen mit einer Stirnseite des Achskörpers eine Dichtungsebene bilden und die zylindermantelförmigen Kontaktflächen eine Dichtungsmantelebene. Dabei kann der dritte Kanalabschnitt durch die Dichtungsebene oder die Dichtungsmantelebene oder im Topfeck durch die Dichtungsebene und die Dichtungsmantelebene verlaufen.

Optional kann die Schwenkachse orthogonal zur Längsrichtung verlaufen. Alternativ dazu kann gemäß einer weiteren beispielhaften Ausführungsform die Schwenkachse in einem Winkel von 40° bis 60° zur Längsrichtung verlaufen. Dadurch kann die Anbindung und Auslegung des zumindest einen Fluidkanals vereinfacht werden.

Optional kann der Instrumentenkopf über den zumindest einen Fluidkanal fluidisch antreibbar und/oder steuerbar sein. Alternativ oder zusätzlich dazu kann der Fluidkanal dazu dienen, eine Spülflüssigkeit und/oder ein Abrasiv-Schneidmittel zum Instrumentenkopf zu führen. Der Abwinklungsgrad des Gelenks kann vorzugsweise über mechanische Steuerelemente wie Zugseile oder Schubstangen gesteuert werden. Alternativ dazu kann der Abwinklungsgrad des Gelenks fluidisch antreibbar und/oder steuerbar sein.

Optional kann der Instrumentenkopf mindestens zwei Freiheitsgrade zur Bewegung bzw. Betätigung aufweisen, wobei das Instrument zum fluidischen Antrieb oder zur fluidischen Steuerung des Instrumentenkopfs für jeden Freiheitsgrad jeweils mindestens einen vom proximalen Schaftabschnitt in den distalen Schaftabschnitt führenden separaten Fluidkanal aufweist. Beispielsweise können die Maulteile einer Zange oder die Klingen einer Schere gemäß einem ersten Freiheitsgrad über einen ersten Fluidkanal fluidisch in Öffnungs- und Schließstellung betätigt werden. Dazu kann beispielsweise ein pneumatischer oder hydraulischer Kolben in Längsrichtung vor und zurück bewegt werden, wobei der Kolben über Hebelarme mit den Maulteilen bzw. Klingen derart mechanisch gekoppelt ist, dass eine Bewegung des Kolbens in Längsrichtung eine Bewegung der Maulteilen bzw. Klingen bewirkt. Die mechanische Arbeit kann mittels Druck und Unterdruck des Fluids verrichtet werden.

Wenn über den Fluidkanal nur mit Druck, aber nicht mit Unterdruck mechanische Arbeit verrichtet werden soll (z.B. vorzugsweise bei pneumatischem Antrieb mit Druckluft), so kann beispielsweise für einen ersten oder zweiten Freiheitsgrad ein Fluidkanalpaar vorgesehen sein, wobei das Fluidkanalpaar einen distalwärtigen Vorlauf und einen proximalwärtigen Rücklauf bereitstellt. Mittels der Durchflussrichtung durch das Fluidkanalpaar kann die Bewegungsrichtung des Freiheitsgrads gesteuert werden. Ein solches Fluidkanalpaar ist insbesondere dann vorteilhaft, wenn eine kontinuierliche Bewegung, wie etwa die kontinuierliche Rotation eines Fräskopfs mittels eines Hydromotors angetrieben werden soll und das Fluid nicht als Spülmittel distalseitig abgelassen werden soll. Die Drehrichtung ist dabei durch die Durchflussrichtung bestimmt. Als Alternative oder zusätzlich zu einem Fluidkanalpaar und einem Druck/Unterdruck-Fluidkanal kann insbesondere bei begrenzten Bewegungen (z.B. einer Klingen- oder Maulteilbewegung) eine der Bewegungsrichtungen durch eine Federspannung erzeugt werden, die bei fluidisch angetriebener Bewegung in die andere Richtung gespannt wird, sodass nur ein Druck-Fluidkanal benötigt wird. Alternativ oder zusätzlich zu einem Kolben kann auch ein fluidischer Muskel über einen der Fluidkanäle betätigt werden, wobei der fluidische Muskel einen druckdichten Schlauch aufweist, der sich bei Druckbeaufschlagung radial weitet und axial verkürzt, sodass damit mechanische Arbeit am Instrumentenkopf verrichtet werden kann.

Optional können die jeweiligen dritten ringförmig um die Schwenkachse verlaufenden Kanalabschnitte der Fluidkanäle parallel zueinander angeordnet sein, wobei jeweils der erste Ringwandungsabschnitt der Fluidkanäle durch eine bezüglich der Schwenkachse radiale Außenfläche des Achskörpers gebildet ist und jeweils der zweite Ringwandungsabschnitt der Fluidkanäle durch eine bezüglich der Schwenkachse radiale Innenfläche des zweiten Gelenkteils gebildet ist. Die oben beschriebenen Ausführungsformen sind daher besonders gut geeignet, mehrere Fluidkanäle durch das Gelenk zu führen.

Optional kann der zumindest eine Fluidkanal zumindest abschnittsweise mittels eines generativen Fertigungsverfahrens für eines oder mehrere Teile des Instruments ausgebildet sein. Insbesondere die oben beschriebenen Ausführungsformen können auf kleinstem Bauraum Abschnitte der Fluidkanäle aufweisen, die in komplexer Weise durch die Gelenkteile verlaufen. Mit einem generativen Fertigungsverfahren (Additive Manufacturing, AM) wie etwa 3D-Druck mit Pulver, Selektivem Lasersintern (SLS), Selektivem Laserschmelzen (SLM), Direktem Metall Lasersintern (DMLS), Elektronenstrahlschmelzen (EBM), Schmelzschichtung (FDM, Fused Deposition Modeling), Laserauftragsschweißen, Multi-Jet Modeling (MJM), Stereolithographie (SL) Poly-Jet, Laminated Object Modeling (LOM), Countour Crafting (CC), Film Transfer Imaging (FTI) oder Digital Light Processing (DLP) können komplexe innere Fluidkanäle auf kleinstem Bauraum für Teile des Instruments aus Plastik, Keramik und/oder Metall realisiert werden.

Optional kann der zumindest eine Fluidkanal eine vom Gelenk proximal angeordnete Fluiddruckquelle mit einem vom Gelenk distal angeordneten hydraulischen Motor fluidverbinden, wobei der hydraulische Motor vorzugsweise nach dem Gerotor-Prinzip, dem Kappelmotorprinzip oder dem Spannungswellengetriebeprinzip aufgebaut ist.

Beim hydraulischen Motor nach dem Gerotor-Prinzip wird ein inneres Zahnrad mit n Zähnen, das exzentrisch auf einer Innenverzahnung einer Hülse mit n+1 Zähnen abrollen kann, fluidisch angetrieben. Das innere Zahnrad bildet mindestens zwei Dichtlinien mit der Innenverzahnung der Hülse und somit zwei Volumenkammern, die sich je nach Drehrichtung periodisch vergrößern bzw. verkleinern. Wird ein Fluidkanalpaar mit einem distalwärtigen Vorlauf mit der ersten Volumenkammer und mit einem proximalwärtigen Rücklauf mit der zweiten Volumenkammer fluidverbunden, kann das innere Zahnrad über einen Durchfluss durch das Fluidkanalpaar angetrieben werden und eine mit dem Instrumentenkopf gekoppelte Welle antreiben. Bei Umkehrung der Durchflussrichtung durch das Fluidkanalpaar wird der distalwärtige Vorlauf zum proximalwärtigen Rücklauf und umgekehrt, sodass sich das innere Zahnrad in die andere Richtung auf der Innenverzahnung der Hülse abrollt.

Alternativ zu einem Fluidkanalpaar mit proximalwärtigem Rücklauf kann der hydraulische Motor nach dem Gerotor-Prinzip nur mit einem Fluidkanal als distalwärtigem Vorlauf über die erste Fluidkammer fluidisch angetrieben werden und die zweite Fluidkammer mit einem distalseitigen Spülauslass verbunden sein. Das Antriebsfluid wird dadurch als Spülfluid verbraucht und nicht in einem Kreislauf gepumpt, in dem die Durchflussrichtung umgekehrt werden kann. Die Drehrichtung ist dann dadurch festgelegt, mit welcher Fluidkammer der Vorlauf fluidisch verbunden ist, und somit nicht umkehrbar.

Das Prinzip des Kappelmotors funktioniert über zwei um ±90° phasenverschobene periodische Schwingungsanregungen eines inneren Antriebsrings aus 90° zueinander versetzten Richtungen, wobei für die Anregungen typischerweise Piezo-Elemente verwendet werden. Gemäß der vorliegenden Offenbarung kann dieses Prinzip auf einen hydraulischen Motor für einen fluidisch antreibbaren bzw. steuerbaren Instrumentenkopf übertragen werden, indem für die unmittelbaren Anregungen keine Piezo-Elemente verwendet werden, sondern Fluidkanäle, die periodisch über eine proximalseitig vorgesehene Fluiddruckquelle bedruckt werden. Der Antriebsring kann eine Innenverzahnung oder einen inneren Reibschluss mit einer verzahnten oder reinschlüssigen Mantelaußenfläche einer konzentrisch in Längsrichtung angeordneten Motorwelle aufweisen. Dabei ist der Durchmesser des Antriebsrings etwas größer als der Durchmesser der Mantelaußenfläche der Motorwelle. Bei einer Verzahnung kann die Innenverzahnung des Antriebsrings einen oder mehr Zähne mehr ausweisen als die Mantelaußenfläche der Motorwelle. Der Antriebsring kann sowohl in einer ersten Querrichtung (x) zur Motorwelle schwingen als auch in einer zur ersten Querrichtung (x) senkrechten zweiten Querrichtung (y) zur Motorwelle. Eine sich in der ersten Querrichtung (x) ausdehnbare erste Fluidkammer kann mit einem ersten Fluidkanal fluidverbunden sein und den Antriebsring fluidisch in der ersten Querrichtung (x) zur Motorwelle zu Schwingungen anregen. Eine sich in der zweiten Querrichtung (y) ausdehnbare zweite Fluidkammer kann mit einem zweiten Fluidkanal fluidverbunden sein und den Antriebsring fluidisch in der zweiten Querrichtung (y) zur Motorwelle zu Schwingungen anregen. Sind die Schwingungen ±90° zueinander phasenverschoben, dreht sich die Motorwelle, die auf der Innenverzahnung des Antriebsrings abrollt. Das Vorzeichen der Phasenverschiebung bestimmt die Drehrichtung, und die Frequenz der Anregungsschwingung bestimmt die Drehgeschwindigkeit. Das Untersetzungsverhältnis zwischen Anregungsschwingung und Drehfrequenz kann durch die Zähnezahldifferenz bzw. die Durchmesserdifferenz eingestellt werden. Die maximale Ausdehnung der Fluidkammern in x- und y-Richtung ist vorteilhafterweise mindestens so groß wie die Durchmesserdifferenz und größer als die Zahnhöhe, um einen kontinuierlichen Zahneingriff bzw. Reibschluss zu gewährleisten. Die Zahnhöhe kann hier im Bereich von einigen 10 Mikrometern liegen. Gegenüber dem Gerotor-Prinzip hat das Kappelmotorprinzip den Vorteil, dass kein kontinuierlicher Fluiddurchfluss mit entsprechendem Reibungsverlust benötigt wird, sondern das Fluid nur Druckwellen überträgt. Der hierin beschriebene hydraulische Motor nach dem Kappelmotorprinzip kann einen Wirkungsgrad von 95% erreichen. Proximalseitig können die Druckwellen mittels Piezo-Elementen in das Fluid eingeleitet werden, sodass der hydraulische Motor nach dem Kappelmotorprinzip mittelbar durch Piezo-Elemente antreibbar sein kann.

Beim hydraulischen Motor nach dem Spannungswellengetriebeprinzip (auch als Harmonic Drive, Wellgetriebe, Gleitkeilgetriebe oder Strainwave gear (SWG) bezeichnet), wälzt sich ein elastisches Übertragungselement (Flexspline) mit Außenverzahnung auf einer etwas größeren Innenverzahnung einer festen Hülse (Circular Spline) ab, wobei sich das Flexspline elliptisch verformen lässt, sodass die Außenverzahnung des Flexsplines im Bereich der großen Ellipsenachse in die Innenverzahnung der Hülse greift und im Bereich der kleinen Ellipsenachse nicht in die Innenverzahnung des Circular Splines greift. Typischerweise rotiert innerhalb des Flexsplines eine von einer Antriebswelle angetriebene elliptische Formscheibe (Wave Generator), die das Flexspline von innen so verformt, dass die große Ellipsenachse und damit die sich gegenüberliegenden Eingriffsbereiche der Verzahnung rotieren. Dabei rotiert das Flexspline gemäß einem durch das Zahnverhältnis zwischen Innen- und Außenverzahnung bestimmten Untersetzungsverhältnis und kann eine Abtriebswelle antreiben, die mit dem Instrumentenkopf gekoppelt sein kann.

Gemäß der vorliegenden Offenbarung kann dieses Prinzip auf einen hydraulischen Motor für einen fluidisch antreibbaren bzw. steuerbaren Instrumentenkopf übertragen werden, indem für die unmittelbaren Verformung des Flexsplines kein Wave Generator in Form einer elliptischen Formscheibe verwendet wird, sondern unmittelbar durch Fluiddruck, der über Fluidkanäle auf das Flexspline ausgeübt wird. Beispielsweise können eine Mehrzahl von Radialkolben oder ausdehnbare Fluidkammern in einem verformbaren Grundkörper als Wave Generator fungieren und derart aus einer Mehrzahl von Fluidkanälen mit Fluiddruck angesteuert werden, dass die sich gegenüberliegenden Eingriffsbereiche der Verzahnung zwischen Flexspline und Circular Spline rotieren und damit das Flexspline angetrieben wird.

Optional kann der zumindest eine Fluidkanal mit mindestens einem vom hydraulischen Motor distalseitig angeordneten Fluidauslass fluidverbunden sein, sodass ein bioverträgliches Fluid, wie etwa isotonische 0,9%ige Salzlösung, zum Antrieb des hydraulischen Motors als Spül- oder Schneidstrahlflüssigkeit verwendet werden kann. Wie bereits oben beschrieben, ist die Verwendung des Antriebsfluids als Spülflüssigkeit besonders in Verbindung mit einem hydraulischen Motor nach dem Gerotor-Prinzip mit nur einem Vorlaufkanal vorteilhaft. Bei Verwendung des bioverträglichen Fluids als Schneidstrahlflüssigkeit wird ein entsprechend hoher Druck benötigt, der mit einem entsprechend hohen Vorlauf- und Rücklaufdruck in einem Fluidkanalpaar bereitgestellt werden kann und vorzugsweise im Rücklauf dem Antriebskreislauf zum Abrasiv-Strahlschneiden entnommen werden kann. Die Menge entnommener Schneidstrahlflüssigkeit kann im Antriebskreislauf proximalseitig kontinuierlich oder diskontinuierlich wieder aufgefüllt werden, um den benötigten Vorlauf- und Rücklaufdruck aufrecht zu erhalten.

Alternativ zu isotonischer Salzlösung kann als steriles bioverträgliches Fluid ein biokompatibles Öl zum Einsatz kommen, um bessere Schmiereigenschaften, höhere Viskosität für höhere Dichtigkeit und/oder eine Korrosionshemmung zu erzielen. Alternativ dazu können auch speziell modifizierte Lösungen zum Einsatz kommen, bei denen beispielsweise die Viskosität durch Zusätze erhöht ist, z.B. eine sterile wässrige Lösung von Polyethylenglycol.

Optional kann ein Über- oder Untersetzungsgetriebe mechanisch zwischen dem hydraulischen Motor und dem Instrumentenkopf gekoppelt sein, wobei das Über- oder Untersetzungsgetriebe vorzugsweise in Form eines wie oben beschriebenen Spannungswellengetriebes ausgestaltet ist. Die Antriebswelle des Spannungswellengetriebes für den Wave Generator in Form einer elliptischen Formscheibe kann durch die Abtriebswelle eines zuvor beschriebenen hydraulischen Motors, der sich proximalseitig vom Getriebe befindet, angetrieben werden. Die Abtriebswelle des Getriebes kann direkt mit dem distalseitigen Instrumentenkopf gekoppelt sein.

Optional kann der mindestens eine Fluidkanal über Entlüftungsventile oder bewusste Leckage durch bestimmt gewählte Spaltmaße entlüftet werden. In diesem Zusammenhang kann das fluidische System des Instruments mit einem Überdruck von beispielsweise bis zu 1 bar betrieben werden, damit Blut oder andere nicht sterile Verunreinigungen nicht in das Instrument eindringen können, sondern das sterile Fluid austritt. Dies hat außerdem den Vorteil, dass die Lageroberflächen bzw. Gelenkflächen als Nassläufer auf einem Fluidfilm laufen können, und somit die Reibung und die Auftrittswahrscheinlichkeit eines unerwünschten Stick-Slip-Effekts reduziert sind. Um bei gewollter oder ungewollter Leckage in jedem Fall einen scharfen Austrittsstrahl zu verhindern, der den Patienten verletzen könnte, können Entlüftungsventile und/oder Dichtlinien in Patientenrichtung durch Hinterschnitte und winklige Absätze verdeckt sein.

Zur Vermeidung von Korrosion und entsprechender Abnutzung und Leckage können die Oberflächen der Teile des Instruments, insbesondere der beweglichen Teile, vorzugsweise hartmetallische oder oxidkeramische Werkstoffe wie etwa Wolframcarbid-Nickel aufweisen. Die mit dem Fluid in Kontakt stehenden Oberflächen weisen vorzugsweise ein korrosionsfestes Material auf, wie etwa Edelstahl oder inerter Kunststoff, und möglichst kein reines Buntmetall. Alternativ oder zusätzlich kann das Instrument eine Opferanode oder Fremdstromanode aufweisen bzw. mit dieser verbindbar sein, sodass die Korrosion der Instrumententeile stark verlangsamt oder gänzlich unterbunden werden kann.

Optional kann das Instrument Sensoren aufweisen oder mit diesen gesteuert und überwacht werden. Beispielsweise kann mittels eines Drucksensors der Druck im Fluidkanal gemessen werden und damit auf die entsprechend erzeugten Kräfte und Drehmomente am Instrumentenkopf geschlossen werden. Solch ein Drucksensor kann im Instrument und/oder in einem proximalseitig an das Instrument gekoppelten Steuerungs- und Versorgungssystem angeordnet sein. Damit lässt sich eine Regelung der Kräfte und Drehmomente am Instrumentenkopf mit Rückkopplung über den Fluiddruck realisieren. In einem solchen proximalseitig an das Instrument gekoppelten Steuerungs- und Versorgungssystem kann eine Fluiddruckquelle und ggf. ein Fluidtank vorgesehen sein.

Alternativ oder zusätzlich zu einem Drucksensor kann mindestens ein elektronischer oder optischer Sensor (z.B. Fiber-Bragg-Sensor) zur Messung des Abwinklungsgrads und/oder der Drehgeschwindigkeit im Instrument vorgesehen sein. Alternativ oder zusätzlich können ein oder mehrere von außen sichtbare und bewegliche Teile des Instruments eine außenseitige Markierung aufweisen, die von einem endoskopischen Bild optisch oder durch Infrarot-Licht erfassbar sind, sodass ein Abwinklungsgrad und/oder eine Drehgeschwindigkeit aus der Position bzw. Bewegung der Markierung im endoskopischen Bild bestimmbar sind. Die Markierung kann eine abgesetzte, reflektierende und/oder fluoreszierende Farbgebung, eine eindeutige Geometrie oder eine charakteristische Oberflächenbeschaffenheit sein. Die endoskopische Kamera kann dabei Teil des Instruments und vorzugsweise lateralseitig am Schaft und proximalseitig vom Gelenk mit distalwärts ausgerichteter Blickrichtung angeordnet sein.

Die Offenbarung ist nachfolgend anhand von in den Zeichnungen dargestellten Ausführungsbeispielen näher erläutert. Es zeigen:

Fig. 1 eine perspektivische Explosionsansicht auf einen distalen Teil einer beispielhaften Ausführungsform des hierin offenbarten Instruments;

Fig. 2 eine halbdurchlässige Draufsicht und eine halbdurchlässige Seitenansicht auf ein demontiertes Gelenk einer ersten beispielhaften Ausführungsform des hierin offenbarten Instruments;

Fig. 3 drei Ansichten auf ein Gelenk einer zweiten beispielhaften Ausführungsform des hierin offenbarten Instruments;

Fig. 4 drei Ansichten auf ein Gelenk einer dritten beispielhaften Ausführungsform des hierin offenbarten Instruments;

Fig. 5 eine Ansicht auf einen Halbschnitt eines Gelenks einer beispielhaften Ausführungsform des hierin offenbarten Instruments;

Fig. 6 eine Seitenansicht auf einen distalen Teil einer beispielhaften Ausführungsform des hierin offenbarten Instruments; und

Fig. 7 eine optionale farbige halbdurchlässige Ansicht auf einen distalen Teil einer beispielhaften Ausführungsform des hierin offenbarten Instruments.

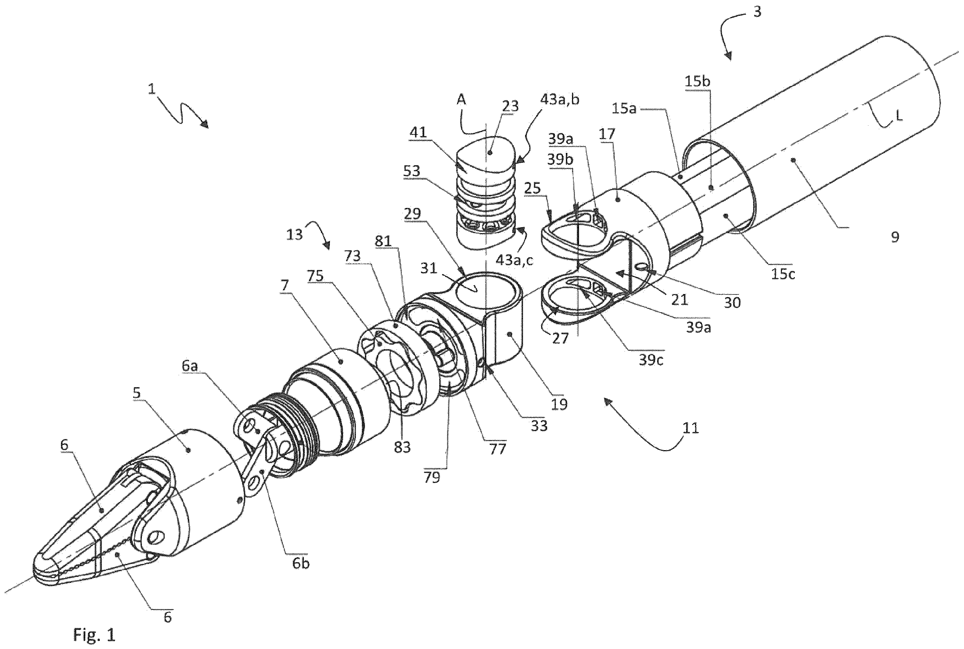

In Fig. 1 ist der distale Teil eines minimalinvasiven medizinischen Instruments 1 gezeigt. Das Instrument weist einen sich in einer Längsrichtung L erstreckenden länglichen Schaft 3 auf. Der Schaft 3 kann proximalseitig mit einem Griff, einer Handhabe, einem Roboterarm und/oder einer anderen maschinellen Führung verbunden sein. Am distalen Ende des Instruments 1 bzw. des Schafts 3 befindet sich ein beweglicher und betätigbarer Instrumentenkopf 5 in Form einer Zange mit Maulteilen 6, die über Hebelarme 6a,b ansteuerbar sind. Der Instrumentenkopf 5 ist mit einem distalen Schaftabschnitt 7 verbunden, der gegenüber einem proximalen Schaftabschnitt 9 mittels eines dazwischen angeordneten Gelenks 11 um eine zur Längsrichtung L senkrecht verlaufende Schwenkachse A abwinkelbar ist. Im distalen Schaftabschnitt 7 ist ein hydraulischer Motor 13 angeordnet, der hier nach dem Gerotor-Prinzip funktioniert, um den Instrumentenkopf 5 zu einer Drehung um die Längsrichtung L fluidisch antreiben zu können. Es sei hierbei angemerkt, dass die Längsrichtung L bei einer Abwinkung des distalen Schaftabschnitt 7 um die Schwenkachse A im distalen Schaftabschnitt 7 mit verschwenkt wird, sodass die Längsrichtung $L_1$ des distalen Schaftabschnitt 7 nicht mehr mit der Längsrichtung $L_2$ des proximalen Schaftabschnitts 9 fluchtet. Bei abgewinkeltem distalem Schaftabschnitt 7 (nicht gezeigt) treibt der hydraulische Motor 13 den Instrumentenkopf 5 zu einer Drehung um die abgewinkelte Längsrichtung $L_1$ des distalen Schaftabschnitts 7 fluidisch an.

Das Instrument 1 weist zudem drei vom proximalen Schaftabschnitt 9 in den distalen Schaftabschnitt führende Fluidkanäle 15a,b,c auf. Die Fluidkanäle 15a,b,c können dabei als separate Leitungen durch den Schaft 3 verlaufen oder als Längskammern des Schafts mit als Trennwänden zwischen den Fluidkanälen 15a,b,c fungierenden Wandungen ausgestaltet sein.

Das Gelenk 11 weist ein erstes Gelenkteil 17 und ein gegenüber dem ersten Gelenkteil 17 um die Schwenkachse A verschwenkbares zweites Gelenkteil 19 auf. Das erste Gelenkteil 17 ist hier proximalseitig vom zweiten Gelenkteil 19 angeordnet und mit dem distalseitigen Ende des proximalen Schaftabschnitts 9 verbunden. Die Gelenkteile 17, 19 könnten allerdings vertauscht sein, d.h. das erste Gelenkteile 17 distalseitig und das zweite Gelenkteil 19 proximalseitig. Das erste Gelenkteil 17 weist hier distalseitig eine maulförmige Aufnahme 21 und einen im Wesentlichen zylindrischen Achskörper 23 auf. Die maulförmige Aufnahme 21 weist dazu in Richtung der Schwenkachse A verlaufende Durchbrechungen 25, 27 auf, durch welche der Achskörper 23 während der Montage des Instruments 1 axial Richtung der Schwenkachse A in die maulförmige Aufnahme 21 eingeführt werden kann.

Die maulförmige Aufnahme 21 des ersten Gelenkteils 17 ist so ausgestaltet, dass sie eine im Wesentlichen rohrförmige und sich in Richtung der Schwenkachse A erstreckende weibliche Aufnahme 29 des zweiten Gelenkteils 19 bezüglich der Schwenkachse A axial umgreift. Die weibliche Aufnahme 29 des zweiten Gelenkteils 19 ist in der maulförmigen Aufnahme 21 des ersten Gelenkteils 17 um die Schwenkachse A drehbar angeordnet. Die weibliche Aufnahme 29 definiert eine bezüglich der Schwenkachse A radiale Innenfläche 31 des zweiten Gelenkteils 19, die mit den Durchbrechungen 25, 27 der maulförmigen Aufnahme 21 des ersten Gelenkteils 17 koaxial zur Schwenkachse A fluchtet, sodass der Achskörper 23 während der Montage des Instruments 1 in Richtung der Schwenkachse A axial durch die Durchbrechungen 25, 27 und die weibliche Aufnahme 29 in die maulförmige Aufnahme 21 eingeführt werden kann. Der eingeführte Achskörper 23 kann dann während der Montage des Instruments 1 mit der maulförmigen Aufnahme 21 des ersten Gelenkteils 17 fest verbunden werden, beispielsweise durch Verkleben, Löten oder Schweißen. Die den Achskörper 23 umgreifende weibliche Aufnahme 29 des zweiten Gelenkteils 19 bleibt dabei gegenüber dem Achskörper 23 um die Schwenkachse A drehbar.

Die Verschwenkung um die Schwenkachse A wird in dieser gezeigten ersten Ausführungsform nicht fluidisch, sondern mechanisch über Zugseile (nicht gezeigt) bewirkt. Die hier nicht gezeigten Zugseile erstrecken sich in Längsrichtung L durch den Schaft 3 und treten jeweils an einer lateralen Seite aus einer Zugseildurchführung 30 im proximalen Gelenkteil 17 aus und sind mit dem distalen Gelenkteil 19 an der jeweiligen lateralen Seite mittels einer Zugseilaufnahme 33 verbunden. Durch Zug an einem der Zugseile kann ein Drehmoment zum Verschwenken um die Schwenkachse A in eine Schwenkrichtung auf das distalen Gelenkteil 19 ausgeübt werden, und entsprechend durch Zug am anderen der Zugseile ein Drehmoment zum Verschwenken des distalen Gelenkteils um die Schwenkachse A in die andere Schwenkrichtung.

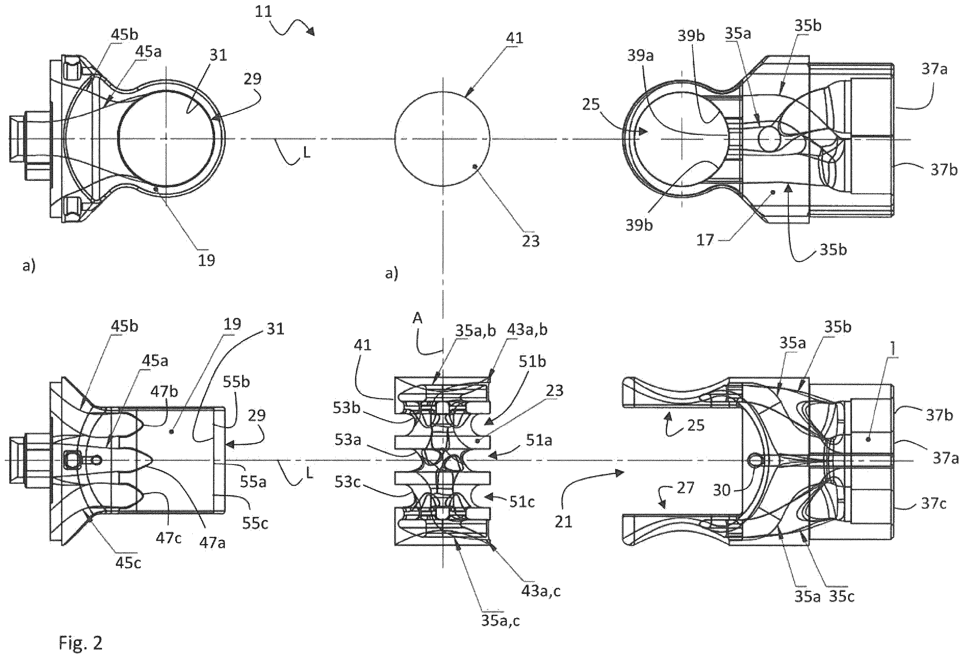

Die Fluidkanäle 15a,b,c weisen jeweils einen ersten Kanalabschnitt 35a,b,c auf, der durch den ersten Gelenkteil 17 verläuft. Die ersten Kanalabschnitte 35a,b,c verlaufen getrennt voneinander geschwungen durch das Innere des ersten Gelenkteils 17 von einem jeweiligen proximalen Ende 37a,b,c (siehe Fig. 2), das mit einem entlang des Schafts 3 verlaufenden jeweiligen proximalen Abschnitt der Fluidkanäle 15a,b,c fluidverbunden ist, zu einem Übergang 39a,b,c, der an einer Innenfläche der Durchbrechungen 25, 27 der maulförmigen Aufnahme 21 des ersten Gelenkteils 17 angeordnet ist. Hier sind die Übergänge 39a,b,c jeweils zweiteilig ausgestaltet, wobei sich die ersten Kanalabschnitte 35a,b,c innerhalb des ersten Gelenkteils 17 jeweils aufspalten. Der erste Kanalabschnitt 35a des ersten Fluidkanals 15a spaltet sich so auf, dass ein Teil in den jeweils mittig angeordneten Übergang 39a beider Durchbrechungen 25, 27 mündet. Der erste Kanalabschnitt 35b des zweiten Fluidkanals 15b spaltet sich so auf, dass jeweils ein Teil links und rechts vom mittigen Übergang 39a des ersten Fluidkanals 15a in die Übergänge 39b in der oberen Durchbrechung 25 mündet. Der erste Kanalabschnitt 35c des dritten Fluidkanals 15c spaltet sich so auf, dass jeweils ein Teil links und rechts vom mittigen Übergang 39a des ersten Fluidkanals 15a in die Übergänge 39c in der unteren Durchbrechung 27 mündet.

Die ersten Kanalabschnitte 35a,b,c verlaufen weiter durch das Innere des Achskörpers 23. Dazu weist der Achskörper 23 an seiner radialen Außenfläche 41 entsprechend positionierte Übergänge 43a,b,c auf, die beim Einsetzen des Achskörpers 23 während der Montage des Instruments 1 in die Durchbrechungen 25, 27 mit den Übergängen 39a,b,c des proximalen Gelenkteils 17 zur fluiddichten passgenauen Anlage kommen.

Analog weisen die Fluidkanäle 15a,b,c jeweils einen zweiten Kanalabschnitt 45a,b,c auf, der durch den zweiten Gelenkteil 19 verläuft (siehe Fig. 2). Die zweiten Kanalabschnitte 45a,b,c verlaufen getrennt voneinander geschwungen durch das Innere des zweiten Gelenkteils 19 von einem jeweiligen Übergang 47a,b,c, der an einer Innenfläche 31 der weiblichen Aufnahme 29 angeordnet ist, zu einem distalen Ende, das mit einer jeweiligen Zuleitung zum Hydromotor 13 distalseitig fluidverbunden ist. Hier sind die Übergänge 47a,b,c jeweils an unterschiedlichen axialen Positionen bezüglich der Schwenkachse A angeordnet. Der Übergang 47a des zweiten Kanalabschnitts 45a des ersten Fluidkanals 15a ist in axialer Mittelage bezüglich der Schwenkachse A zwischen dem oberen Übergang 47b des zweiten Kanalabschnitts 45b des zweiten Fluidkanals 15b und dem unteren Übergang 47c des zweiten Kanalabschnitts 45c des dritten Fluidkanals 15c angeordnet. Die ersten Kanalabschnitte 35a,b,c verlaufen daher gegenüber den jeweiligen zweiten Kanalabschnitten 45a,b,c bezüglich der Längsrichtung zumindest streckenweise radial versetzt zueinander. Innerhalb des Achskörpers 23 erstrecken sich daher die Fluidkanäle im Wesentlichen in Richtung der Schwenkachse A.

Zwischen dem ersten Kanalabschnitt 35a,b,c und dem zweiten Kanalabschnitt 45a,b,c weist der jeweilige Fluidkanal 15a,b,c einen ringförmigen dritten Kanalabschnitt 51a,b,c auf. Dieser dritte Kanalabschnitt 51a,b,c weist einen vom ersten Gelenkteil 17 bzw. dem Achskörper 23 gebildeten ersten Ringwandungsabschnitt 53a,b,c und einen vom zweiten Gelenkteil 19 bzw. der weiblichen Aufnahme 29 gebildeten zweiten Ringwandungsabschnitt 55a,b,c auf. Der Achskörper 23 erstreckt sich in Richtung der Schwenkachse A durch die ringförmigen dritten Kanalabschnitte 51a,b,c, bzw. bildet er als Teil des ersten Gelenkteils 17 mit seiner radialen Außenfläche 41 die ersten Ringwandungsabschnitte 53a,b,c der dritten Kanalabschnitte 51a,b,c.

In dieser in Fig. 1 und 2 gezeigten ersten Ausführungsform grenzen der erste Ringwandungsabschnitt 53a,b,c und der zweite Ringwandungsabschnitt 55a,b,c in radialer Richtung bezüglich der Schwenkachse A aneinander an. Der erste Ringwandungsabschnitt 53a,b,c bildet einen Innenteil der Wandung und der zweite Ringwandungsabschnitt 55a,b,c einen Außenteil. Der Achskörper 23 als Teil des ersten Gelenkteils 17 und die weibliche Aufnahme 29 als Teil des zweiten Gelenkteils 19 weisen jeweils im Wesentlichen zylindermantelförmige Kontaktflächen 31, 41 auf, die in einer koaxial zur Schwenkachse A verlaufenden Dichtungsmantelebene um die Schwenkachse A herum dichtend radial aneinander liegen. Wegen der Rotationsymmetrie der dritten Kanalabschnitte 51a,b,c ist der Bewegungsspielraum des Gelenks 11 und damit die Bandbreite von Anwendungsmöglichkeiten des Instruments 1 nicht durch den Fluidkanal 15a,b,c beschränkt. Außerdem ist das zum Abwinkein benötigte Drehmoment im Wesentlichen unabhängig vom Druck im Fluidkanal 15a,b,c. Schließlich ist der Querschnitt des Fluidkanals 15a,b,c bei jedem Grad der Abwinklung gleich.

In Fig. 2 ist das Gelenk 11 in dieser ersten Ausführungsform genauer mit seinen Bestandteilen gezeigt. Fig. 2 zeigt dabei oben eine Draufsicht auf das zweite distalseitige Gelenkteil 19, das die weibliche Aufnahme 29 mit im Wesentlichen zylindrischer radialer Innenfläche 31 definiert. Fig. 2 zeigt oben auch eine Draufsicht auf den Achskörper 23 des ersten proximalseitigen Gelenkteils 17. Der Achskörper 23 ist korrespondierend zur radialen Innenfläche 31 der weiblichen Aufnahme 29 des zweiten Gelenkteils 19 im Wesentlichen zylindrisch mit der radialen Außenfläche 41 aufgebaut, die in dichtend gleitendem Kontakt mit der Innenfläche 31 steht. Die Außenfläche 41 und die Innenfläche 31 bilden also im Wesentlichen zylindermantelförmige Kontaktflächen, die in einer koaxial zur Schwenkachse A verlaufenden Dichtungsmantelebene um die Schwenkachse A herum dichtend radial aneinander liegen. Die Durchbrechungen 27, 29 der maulförmigen Aufnahme 21 des ersten Gelenkteils 17 weisen in der in Fig. 2 oben gezeigten Draufsicht radiale Innenflächen auf, die analog zur Innenfläche 31 eine passgenaue Aufnahme des Achskörpers 23 erlauben. Allerdings wird der Achskörper 23 bei der Montage des Instruments 1 fest mit der maulförmigen Aufnahme 21 des ersten Gelenkteils 17 verbunden. Bei der Montage des Instruments 1 wird zunächst die weibliche Aufnahme 29 des zweiten Gelenkteils 19 derart in die maulförmige Aufnahme 21 des ersten Gelenkteils 17 platziert, dass die jeweiligen Durchbrechungen 27, 29 und die radiale Innenfläche 31 koaxial zur Schwenkachse A fluchten. Die weibliche Aufnahme 29 ist dann bezüglich der Schwenkachse A durch die maulförmige Aufnahme 21 axial fixiert. Sodann wird der Achskörper 23 des ersten Gelenkteils 17 koaxial zur Schwenkachse A durch die Durchbrechungen 27, 29 sowie die weibliche Aufnahme 29 eingeführt und oben und unten mit den Durchbrechungen 27, 29 fest verbunden, beispielsweise durch Verkleben. Dabei kommen die Übergänge 39a,b,c an den Innenflächen der Durchbrechungen 25, 27 zur Anlage mit den Übergängen 43a,b,c des Achskörpers 23 , sodass sich die ersten Kanalabschnitte 35a,b,c vom Inneren des ersten Gelenkteils 17 in den Achskörper 23 erstrecken.

Fig. 2 zeigt unten halbdurchlässige Seitenansichten auf das zweite distalseitige Gelenkteil 19, den Achskörper 23 und das erste Gelenkteil 19. Die zweiten Kanalabschnitte 45a,b,c weisen jeweils in einem axialen Abstand bezüglich der Schwenkachse A zueinander einen Übergang 47a,b,c zur Innenfläche 31 der weiblichen Aufnahme 29 auf. Die Innenfläche 31 der weiblichen Aufnahme 29 bildet den zweiten Ringwandungsabschnitt 55a,b,c, der den radial äußeren Teil der Wandungen der dritten Kanalabschnitte 51a,b,c formt. Innseitig sind die dritten Kanalabschnitte 51a,b,c jeweils in Form eines ersten Ringwandungsabschnitts 53a,b,c durch umlaufende Nuten in der radialen Außenfläche 41 des Achskörpers 23 gebildet. Die jeweils durch das Innere des Achskörpers 23 verlaufenden ersten Kanalabschnitte 35a,b,c münden in die jeweilige umlaufende Nut in der radialen Außenfläche 41 des Achskörpers 23. Somit kann ein Fluid vom ersten Kanalabschnitt 35a,b,c in den dritten Kanalabschnitt 51a,b,c und weiter in den zweiten Kanalabschnitt 45a,b,c in drei separaten Fluidkanälen 15a,b,c durch das Gelenk 11 fließen. Wenngleich hier nicht gezeigt, so kann das Gelenk 11 ein um die Schwenkachse A verlaufendes ringförmiges Dichtelement aufweisen, das zwischen dem ersten Gelenkteil 17 bzw. dem Achskörper 23 und dem zweiten Gelenkteil 19 dichtend angeordnet ist.

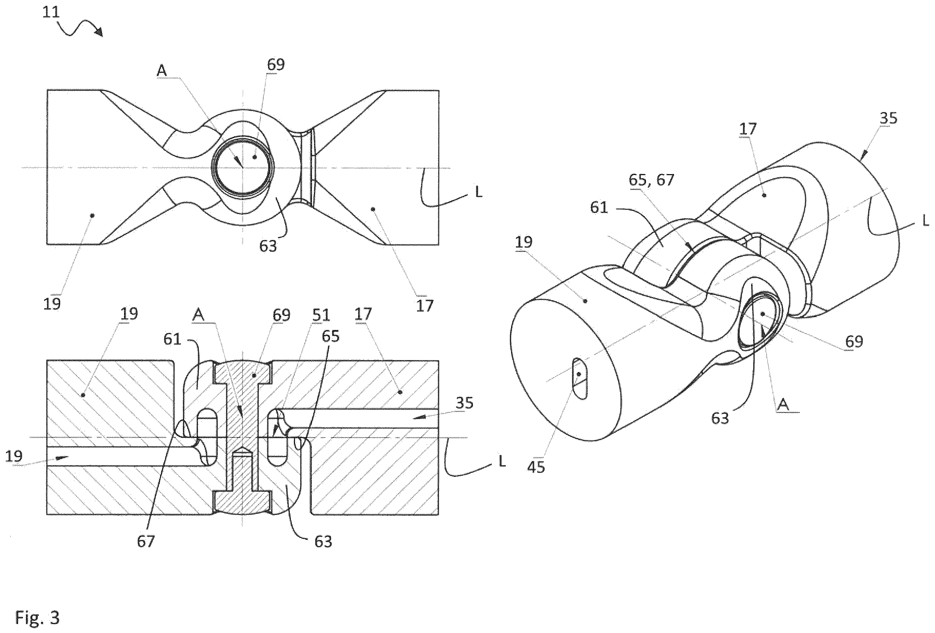

Fig. 3 zeigt drei Ansichten auf eine zweite Ausführungsform des Gelenks 11, wobei oben links eine Draufsicht, rechts ein Querschnitt und unten links eine perspektivische Ansicht dargestellt sind. Hier sind die Gelenkteile 17, 19 im Wesentlichen symmetrisch bzw. identisch zueinander aufgebaut. Die Gelenkteile 17, 19 bilden hier jeweils korrespondierende Gelenkbacken 61, 63 auf, die im Wesentlichen planare Kontaktflächen 65, 67 bilden, die in einer zur Schwenkachse A orthogonalen Dichtungsebene um die Schwenkachse A herum dichtend aneinander liegen. Der Achskörper 23 wird hier durch einen Stift 69 gebildet, der koaxial zur Schwenkachse A durch die Gelenkbacken 61, 63 geführt ist und diese in dichtendem Kontakt zusammenhält. Die Gelenkteile 17, 19 sind hier sowohl gegeneinander als auch gegenüber dem Stift 69 um die Schwenkachse A drehbar. Allerdings könnte der Stift 69 auch fest mit dem einen Gelenkteile 17 oder dem anderen Gelenkteil 19 drehfest verbunden sein. In dieser zweiten Ausführungsform führt nur ein Fluidkanal 15 durch das Gelenk 11, wobei sich auch hier der Fluidkanal 15 zumindest teilweise in Richtung der Schwenkachse A erstreckt, und zwar im Bereich der Gelenkbacken 61, 63. Ein erster Kanalabschnitt 35 verläuft im Inneren des ersten Gelenkteils 17 in die zugehörige Gelenkbacke 61. Ein zweiter Kanalabschnitt 45 verläuft im Inneren des zweiten Gelenkteils 19 ebenfalls in die zugehörige Gelenkbacke 63, und zwar bezüglich der Längsrichtung L radial versetzt. Ein dritter Kanalabschnitt 51 erstreckt sich ringförmig um die Schwenkachse A und verbindet den ersten Kanalabschnitt 35 mit dem zweiten Kanalabschnitt 45. Der ringförmige dritte Kanalabschnitt 51 wird oberseitig durch einen ersten Ringwandungsabschnitt 53 der ersten Gelenkbacke 61 gebildet und unterseitig durch einen zweiten Ringwandungsabschnitt 55 der zweiten Gelenkbacke 63.

Der als Achskörper 23 fungierende Stift 69 erstreckt sich also in Richtung der Schwenkachse A durch den ringförmigen dritten Kanalabschnitt 51. Der erste Ringwandungsabschnitt 53 und der zweite Ringwandungsabschnitt 55 grenzen in axialer Richtung der Schwenkachse A aneinander an und bilden somit den ringförmigen dritten Kanalabschnitt 51. Das Gelenk 11 kann dabei (allerdings nicht gezeigt) ein um die Schwenkachse A verlaufendes ringförmiges Dichtelement aufweisen, das zwischen der ersten Gelenkbacke 61 und der zweiten Gelenkbacke 63 dichtend angeordnet ist. Solch ein Dichtelement kann radial innen vom ringförmigen dritten Kanalabschnitt 51 und/oder radial außen vom ringförmigen dritten Kanalabschnitt 51 in der Dichtungsebene liegen, um den ringförmigen dritten Kanalabschnitt 51 radial abzudichten.

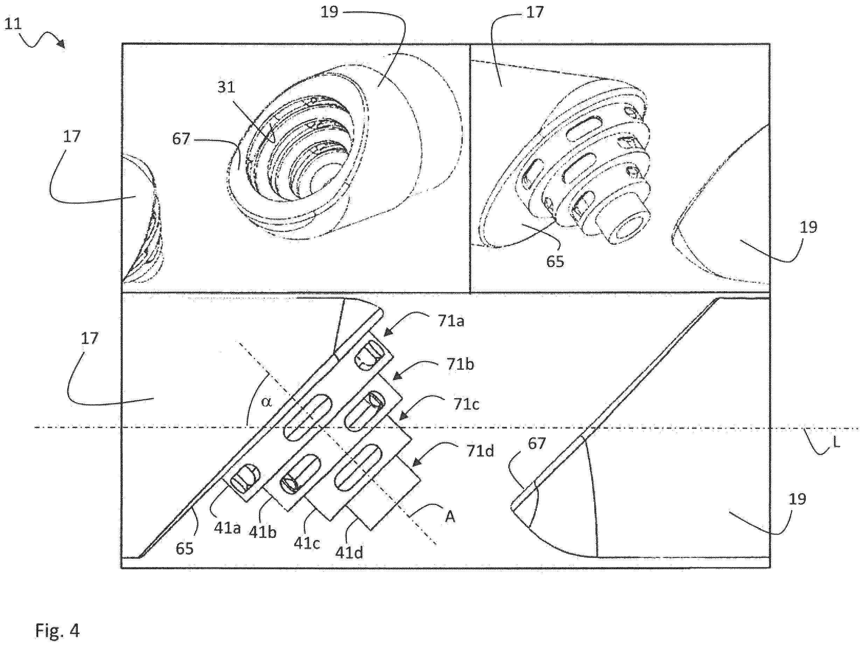

Eine dritte Ausführungsform ist in Fig. 4 gezeigt. Anders als in den vorhergehend beschriebenen zwei Ausführungsformen verläuft in dieser dritten Ausführungsform die Schwenkachse A nicht orthogonal zur Längsrichtung L, sondern in einem Winkel a von 40° bis 60° zur Längsrichtung, hier in einem Winkel a von ca. 45°. Die Verschwenkbarkeit ist hier nur bis zu einem Maximum von 2a, also hier 90° möglich. Der Winkel a sei in diesem Zusammenhang als der spitze Winkel zwischen Schwenkachse A und Längsrichtung L zu verstehen und liegt damit definitionsgemäß im offenen Intervall

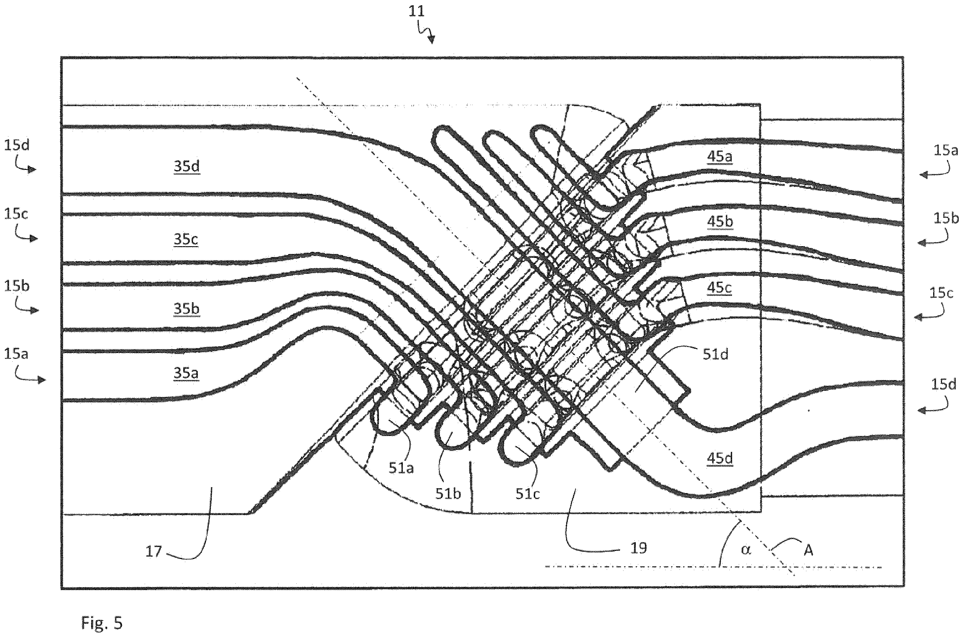

$$]\alpha[ := \{\alpha \in \mathbb{R} \mid 0° < \alpha < 90°\}.$$ Bei Verdrehung des zweiten Gelenkteils 19 gegenüber dem ersten Gelenkteil 17 bewegt sich das zweite Gelenkteil 19 entlang einer Kegelfläche mit halbem Kegelwinkel a um die Schwenkachse A. Diese dritte Ausführungsform ist in gewisser Hinsicht eine Mischung aus den zuvor beschriebenen Ausführungsformen, da hier die Gelenkteile 17, 19 sowohl radiale Kontaktflächen 31, 41 als auch planare Kontaktflächen 65, 67 zueinander aufweisen. Das erste Gelenkteil 17 weist dazu einen sich in Richtung der Schwenkachse A erstreckenden Achskörper 23 auf, der aus koaxial zur Schwenkachse A angeordneten Scheibenabschnitten 71 a,b,c,d aufgebaut ist. Der am nächsten zum Gelenkteil 17 gelegene Scheibenabschnitt 71a hat dabei einen größeren Radius als der drauffolgende Scheibenabschnitt 71b, der wiederum einen größeren Radius hat als der drauffolgende Scheibenabschnitt 71c, der wiederum einen größeren Radius hat als der drauffolgende Scheibenabschnitt 71d. Damit ergibt sich ein bezüglich der Schwenkachse A im Wesentlichen rotationssymmetrischer und treppenartiger Aufbau des Achskörpers 23. Korrespondierend dazu weist das zweite Gelenkteil 19 als weibliche Aufnahme 29 eine bezüglich der Schwenkachse A im Wesentlichen rotationssymmetrische und treppenartige Vertiefung auf, in die der Achskörpers 23 genau und axial sowie radial dichtend hinein passt. Die vier Scheibenabschnitte 71a-d dienen hier dazu, die jeweils dritten Kanalabschnitte 51a-d von vier Fluidkanälen 15a-d zu bilden. Der dritte Kanalabschnitt 51d des vierten Fluidkanals 15d verläuft hier allerdings nicht ringförmig um die Schwenkachse A, sondern in Richtung der Schwenkachse A durch den Achskörper 23. Der Übergang vom zugehörigen ersten Kanalabschnitt 35d in den dritten Kanalabschnitt 51d ist daher axial am kleinsten Scheibenabschnitt 71d des Achskörpers 23 angeordnet. Die anderen Übergänge vom zugehörigen ersten Kanalabschnitt 35a-c in den jeweiligen ringförmigen dritten Kanalabschnitt 51a-c sind auf der radialen Außenfläche 41a-c der entsprechenden Scheibenabschnitte 71a-c des Achskörpers 23 angeordnet. Im Querschnitt von Fig. 5 wird der Verlauf der vier Fluidkanäle 15a-d durch das Gelenk 11 gemäß der dritten Ausführungsform deutlich.

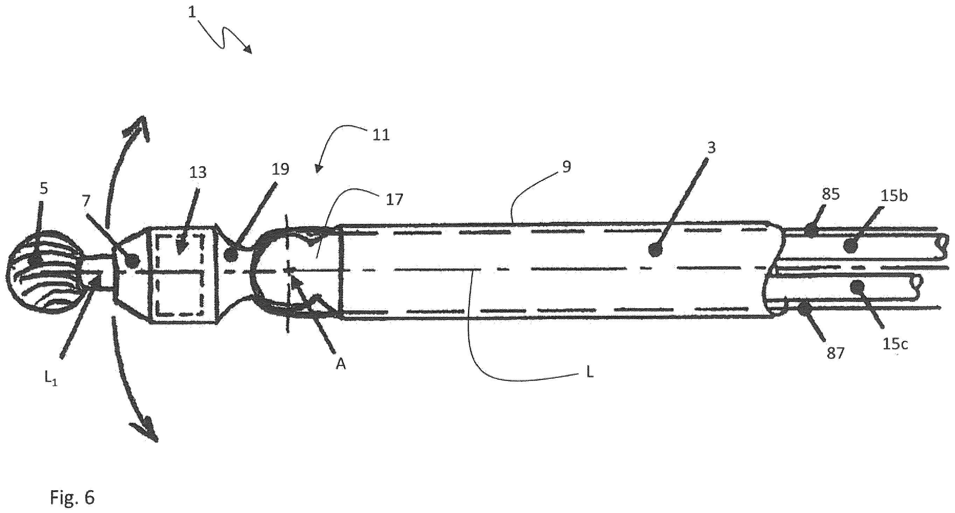

Fig. 6 zeigt einen distalen Anschnitt eines weiteren Ausführungsbeispiels für das hierin offenbarte Instrument 1, wobei hier der Instrumentenkopf 5 einen Fräser aufweist, der um seine Längsachse $L_1$ drehbar an einem über das Gelenk 11 abwinkelbaren distalen Schaftabschnitt 7 angeordnet ist. Im distalen Schaftabschnitt 7 ist zwischen dem Gelenk 11 und dem Fräser ein hydraulischer Motor 13 angeordnet, der hier nach dem Gerotor-Prinzip funktioniert, um den Fräser zu einer Drehung um seine Längsrichtung $L_1$ fluidisch antreiben zu können. Das Gelenk 11 kann gemäß einer der zuvor beschriebenen Ausführungsformen ausgestaltet sein, wobei mindestens ein Fluidkanal 15b,c durch das Gelenk 11 führt, um Fluiddruck von dem durch den Schaft 3 führenden Vorlaufkanal durch das Gelenk 11 zum hydraulischen Motor 13 zu führen und gleichzeitig eine Abwinklung des Gelenks 11 zu erlauben. Hier ist auch ein Rücklaufkanal 15c,b durch den Schaft 3 und das Gelenk 11 vorgesehen, um einen Kreislauf für das Antriebsfluid bereitzustellen. Alternativ oder zusätzlich zu einem Rücklauf könnte das vorzugsweise bioverträgliche Antriebsfluid nach Verrichtung der mechanischen Arbeit im hydraulischen Motor 13 als Spülflüssigkeit distalseitig vom Motor 13 ausgelassen werden. Die Abwinklung des Gelenks 11 um die Schwenkachse A wird über lateral an diametral gegenüberliegenden Seiten, deren Verbindungslinie senkrecht zur Schwenkachse A verläuft, in Längsrichtung L durch den Schaft 3 verlaufende Zugseile 85, 87 bewirkt. Die Zugseile 85, 87 greifen distalseitig kraft- und/oder reibschlüssig am zweiten Gelenkteil 19 an und können, wie hier gezeigt, als zwei Abschnitte eines um die Schwenkachse A umlaufenden Zugseils ausgestaltet sein.

Zum besseren Verständnis der Funktionsweise des fluidisch angetriebenen hydraulischen Motors 13 nach dem Gerotor-Prinzip in es sinnvoll, die Fig. 1 noch einmal zu betrachten. Der Motor 13 weist dazu eine Außenhülse 73 mit zykloidischer Innenverzahnung und ein sich auf der Innenverzahnung der Außenhülse 73 abrollendes inneres Antriebsrad 75 mit entsprechender zykloidischer Außenverzahnung auf. Das innere Antriebsrad 75 hat einen kleineren Außendurchmesser als der Innendurchmesser der Außenhülse 73 und ist exzentrisch bezüglich der Außenhülse 73 angeordnet. Die Außenverzahnung des Antriebsrads 75 hat weniger Zähne, vorzugsweise einen Zahn weniger, als die Innenverzahnung der Außenhülse 73. Dadurch ergeben sich im radialen Zwischenraum zwischen der Außenhülse 73 und dem Antriebsrad 75 immer zwei in Wesentlichen voneinander getrennte Volumenkammern, die sich phasenverschoben zueinander periodisch vergrößern und verkleinern, wenn das Antriebsrad 75 in der Außenhülse 73 exzentrisch abrollt. In einer Ausgangsstellung des Antriebsrads 75 sind die beiden Volumenkammern gleich groß. In Fig. 1 mündet ein nierenförmiger Fluidausgang 77 eines Vorlaufkanals, beispielsweise des Fluidkanals 15b, in die erste dieser beiden in der Neutralstellung gleich großen Volumenkammern. Ein nierenförmiger Fluideingang 79 eines Rücklaufkanals, beispielsweise des Fluidkanals 15c, mündet nierenförmig in die zweite dieser beiden in der Neutralstellung gleich großen Volumenkammern. Der nierenförmige Fluidausgang 77 sowie der nierenförmige Fluideingang 79 sind hier von der distalen Seite 81 des zweiten Gelenkteils 19 gebildet.

Sobald ein Fluiddurchfluss durch den Vorlauf 15b und den Rücklauf 15c fließt, wird das Antriebsrad 75 angetrieben, auf der Außenhülse 73 abzurollen. Innen weist das Antriebsrad 75 einen Innendurchmesser auf, der größer als der Außendurchmesser einer mit der Außenhülse 73 konzentrischen Abtriebswelle 83 ist. Das Antriebsrad 75 ist innerseitig im Reibschluss mit der Abtriebswelle 83 und treibt diese zur Drehung an, wenn das Antriebsrad 75 exzentrisch rotiert. Alternativ zum Reibschluss kann auch hier eine entsprechende Verzahnung zwischen Abtriebswelle 83 und dem Antriebsrad 75 vorgesehen sein. Die Durchmesserverhältnisse bzw. Zahnverhältnisse bestimmen hierbei das Übersetzungsverhältnis, d.h. wie viele Umdrehung das Antriebsrad 75 machen muss, um die Abtriebswelle 83 zur einer Umdrehung anzutreiben. Die Durchflussrichtung bestimmt hierbei die Drehrichtung. Das heißt, dass die Drehrichtung durch Umkehren der Durchflussrichtung bzw. Vertauschen der Funktionen der Fluidkanäle 15b,c als Vorlauf bzw. Rücklauf umgekehrt werden kann.

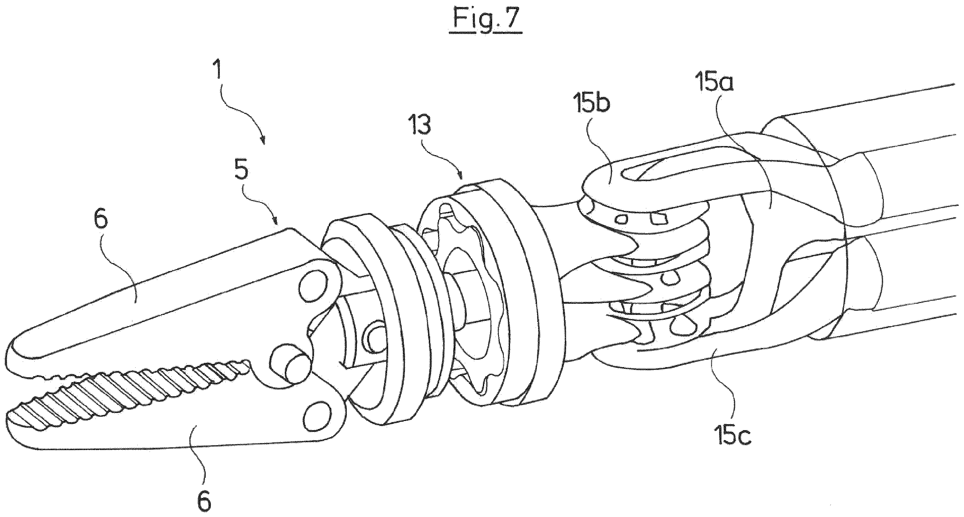

Fig. 7 ist optional gezeigt zum einfacheren Verständnis der oben in Verbindung mit Figuren 1 und 2 beschriebenen Fluiddurchführung durch das Gelenk 11 mit farbig unterschiedlich dargestellten Fluidkanälen 15a,b,c. Über den Fluidkanal 15a als Druck- und Saugleitung wird das Öffnen und Schließen der Zangenmaulteile 6 des Instrumentenkopfs 5 mittels eines in Längsrichtung verschiebbaren Kolbens bewirkt. Der Fluidkanal 15a erstreckt sich dazu konzentrisch in Längsrichtung durch eine hohle Abtriebswelle des hydraulischen Motors 13 zum Instrumentenkopf 5. Die Fluidkanäle 15b und 15c wirken je nach Drehrichtung wie zuvor beschrieben als Vorlauf bzw. Rücklauf für den hydraulischen Motor 13 nach dem Gerotor-Prinzip. Anders als in Figur 6 wird hier nicht ein Fräskopf um seine Längsachse gedreht, sondern der Instrumentenkopf 5 mit den Zangenmaulteilen 6. Die Abwinklung des Gelenks 11 um die Schwenkachse A wird über nicht gezeigte Zugseile bewirkt.

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden.

Äquivalente Ausführungsformen der hierin beschriebenen Parameter, Bauteile oder Funktionen, die in Anbetracht dieser Beschreibung einer fachlich versierten Person als offensichtlich erscheinen, seien hierin so erfasst als wären sie explizit beschrieben. Als optional, vorteilhaft, bevorzugt, erwünscht oder ähnlich bezeichnete "kann"-Merkmale sind als optional zu verstehen und nicht als schutzbereichsbeschränkend.

Die beschriebenen Ausführungsformen sind als illustrative Beispiele zu verstehen und stellen keine abschließende Liste von möglichen Ausführungsformen dar. Jedes Merkmal, das im Rahmen einer Ausführungsform offenbart wurde, kann allein oder in Kombination mit einem oder mehreren anderen Merkmalen verwendet werden, unabhängig davon, in welcher Ausführungsform die Merkmale jeweils beschrieben wurden. Während mindestens ein Ausführungsbeispiel hierin beschrieben und gezeigt ist, seien Abwandlungen und alternative Ausführungsformen, die einer fachmännisch versierten Person in Anbetracht dieser Beschreibung als offensichtlich erscheinen, in dieser Offenbarung mit erfasst. Im Übrigen soll hierin weder der Begriff "aufweisen" zusätzliche andere Merkmale oder Verfahrensschritte ausschließen noch soll "ein" oder "eine" eine Mehrzahl ausschließen.

**Patentansprüche**

1. Minimalinvasives medizinisches Instrument (1) mit

- einem sich in einer Längsrichtung (L) erstreckenden Schaft (3),
- einem sich an einem distalen Ende des Schafts (3) befindlichen beweglichen und/oder betätigbaren Instrumentenkopf (5),
- einem proximalen Schaftabschnitt (9) und einem distalen Schaftabschnitt (7), wobei der distale Schaftabschnitt (7) gegenüber dem proximalen Schaftabschnitt (9) über ein Gelenk (11) um eine Schwenkachse (A) abwickelbar ist, und
- zumindest einem vom proximalen Schaftabschnitt (9) in den distalen Schaftabschnitt (7) führenden Fluidkanal (15),

wobei das Gelenk (11) ein erstes Gelenkteil (17) und ein gegenüber dem ersten Gelenkteil (17) um die Schwenkachse (A) verschwenkbares zweites Gelenkteil (19) aufweist, wobei der zumindest eine Fluidkanal (15) einen ersten Kanalabschnitt (35) im ersten Gelenkteil (17), einen zweiten Kanalabschnitt (45) im zweiten Gelenkteil (19) und einen sich ringförmig um die Schwenkachse (A) erstreckenden dritten Kanalabschnitt (51) aufweist, **dadurch gekennzeichnet, dass** der dritte Kanalabschnitt (51) einen ersten vom ersten Gelenkteil (17) gebildeten Ringwandungsabschnitt (53) und einen zweiten vom zweiten Gelenkteil (19) gebildeten Ringwandungsabschnitt (55) aufweist.

2.  Minimalinvasives medizinisches Instrument (1) nach Anspruch 1, wobei sich der zumindest eine Fluidkanal (15) zumindest teilweise in Richtung der Schwenkachse (A) erstreckt.

3.  Minimalinvasives medizinisches Instrument (1) nach Anspruch 1 oder 2, wobei das Gelenk (11) einen Achskörper (23) aufweist, der sich in Richtung der Schwenkachse (A) und durch den ringförmigen Kanalabschnitt (51) erstreckt.

4.  Minimalinvasives medizinisches Instrument (1) nach einem der vorhergehenden Ansprüche, wobei der erste Kanalabschnitt (35) gegenüber dem zweiten Kanalabschnitt (45) bezüglich der Längsrichtung (L) radial zumindest streckenweise versetzt zueinander verläuft.

5.  Minimalinvasives medizinisches Instrument (1) nach einem der vorhergehenden Ansprüche, wobei der erste Ringwandungsabschnitt (53) und der zweite Ringwandungsabschnitt (55) in axialer Richtung der Schwenkachse (A) aneinander angrenzen.

6.  Minimalinvasives medizinisches Instrument (1) nach einem der vorhergehenden Ansprüche, wobei das erste Gelenkteil (17) und das zweite Gelenkteil (19) jeweils im Wesentlichen planare Kontaktflächen (65, 67) aufweisen, die in einer zur Schwenkachse (A) orthogonalen Dichtungsebene um die Schwenkachse (A) herum dichtend aneinander liegen.

7.  Minimalinvasives medizinisches Instrument (1) nach einem der vorhergehenden Ansprüche, wobei das Gelenk (11) ein um die Schwenkachse (A) verlaufendes ringförmiges Dichtelement aufweist, das zwischen dem ersten Gelenkteil (17) und dem zweiten Gelenkteil (19) dichtend angeordnet ist.

8.  Minimalinvasives medizinisches Instrument (1) nach einem der vorhergehenden Ansprüche, wobei der erste Ringwandungsabschnitt (53) und der zweite Ringwandungsabschnitt (55) in radialer Richtung bezüglich der Schwenkachse (A) aneinander angrenzen.

9.  Minimalinvasives medizinisches Instrument (1) nach Anspruch 8, wobei das erste Gelenkteil (17) und das zweite Gelenkteil (19) jeweils im Wesentlichen zylindermantelförmige Kontaktflächen (31, 41) aufweisen, die in einer koaxial zur Schwenkachse (A) verlaufenden Dichtungsmantelebene um die Schwenkachse (A) herum dichtend radial aneinander liegen.

10.  Minimalinvasives medizinisches Instrument (1) nach Anspruch 8 oder 9, wobei das Gelenk (11) einen Achskörper (23) aufweist, der sich in Richtung der Schwenkachse (A) und durch den ringförmigen Kanalabschnitt (51) erstreckt, wobei der Achskörper (23) einen Teil des ersten Gelenkteils (17) bildet und drehfest mit diesem verbunden ist.

11.  Minimalinvasives medizinisches Instrument (1) nach Anspruch 10, wobei ein Teil des ersten Kanalabschnitts (35) durch den Achskörper (23) verläuft.

12.  Minimalinvasives medizinisches Instrument (1) nach einem der Ansprüche 10 bis 11, wobei der erste Ringwandungsabschnitt (53) durch eine bezüglich der Schwenkachse (A) radiale Außenfläche (41) des Achskörpers (23) gebildet ist.

13.  Minimalinvasives medizinisches Instrument (1) nach einem der Ansprüche 8 bis 12, wobei der zweite Ringwandungsabschnitt (55) durch eine bezüglich der Schwenkachse (A) radiale Innenfläche (31) des zweiten Gelenkteils (19) gebildet ist.

14.  Minimalinvasives medizinisches Instrument (1) nach einem der Ansprüche 8 bis 13, wobei die Schwenkachse (A) orthogonal zur Längsrichtung (L) verläuft.

15.  Minimalinvasives medizinisches Instrument (1) nach einem der Ansprüche 8 bis 13, wobei die Schwenkachse (A) in einem Winkel (a) von 40° bis 60° zur Längsrichtung (L) verläuft.

16. Minimalinvasives medizinisches Instrument (1) nach einem der vorhergehenden Ansprüche, wobei der Instrumentenkopf (5) über den zumindest einen Fluidkanal (15) fluidisch antreibbar und/oder steuerbar ist.

17. Minimalinvasives medizinisches Instrument (1) nach einem der vorhergehenden Ansprüche, wobei der Instrumentenkopf (5) mindestens zwei Freiheitsgrade zur Bewegung bzw. Betätigung aufweist, und wobei das Instrument (1) zum fluidischen Antrieb oder zur fluidischen Steuerung des Instrumentenkopfs (5) für jeden Freiheitsgrad jeweils mindestens einen vom proximalen Schaftabschnitt (9) in den distalen Schaftabschnitt (7) führenden separaten Fluidkanal (15a,b,c) aufweist.

18. Minimalinvasives medizinisches Instrument (1) nach Anspruch 17, wobei die jeweiligen dritten ringförmig um die Schwenkachse (A) verlaufenden Kanalabschnitte (51a,b,c) der Fluidkanäle (15a,b,c) parallel zueinander angeordnet sind, wobei jeweils der erste Ringwandungsabschnitt (53a,b,c) der Fluidkanäle (15a,b,c) durch eine bezüglich der Schwenkachse (A) radiale Außenfläche (41) des Achskörpers (23) gebildet ist und jeweils der zweite Ringwandungsabschnitt (55a,b,c) der Fluidkanäle (15a,b,c) durch eine bezüglich der Schwenkachse (A) radiale Innenfläche (31) des zweiten Gelenkteils (19) gebildet ist.

19. Minimalinvasives medizinisches Instrument (1) nach einem der vorhergehenden Ansprüche, wobei der zumindest eine Fluidkanal (15) zumindest abschnittsweise mittels eines generativen Fertigungsverfahrens für eines oder mehrere Teile des Instruments (1) ausgebildet ist.

20. Minimalinvasives medizinisches Instrument (1) nach einem der vorhergehenden Ansprüche, wobei der zumindest eine Fluidkanal (15) eine vom Gelenk (11) proximal angeordnete Fluiddruckquelle mit einem vom Gelenk (11) distal angeordneten hydraulischen Motor (13) fluidverbindet, wobei der hydraulische Motor (13) vorzugsweise nach dem Gerator-Prinzip, nach dem Kappelmotorprinzip oder dem Spannungswellengetriebeprinzip aufgebaut ist.

21. Minimalinvasives medizinisches Instrument (1) nach Anspruch 20, wobei der zumindest eine Fluidkanal (15) mit mindestens einem vom hydraulischen Motor (13) distalseitig angeordneten Fluidauslass fluidverbunden ist, sodass ein bioverträgliches Fluid zum Antrieb des hydraulischen Motors (13) als Spül- oder Schneidstrahlflüssigkeit verwendet werden kann.

22. Minimalinvasives medizinisches Instrument (1) nach Anspruch 20 oder 21, wobei ein Über- oder Untersetzungsgetriebe mechanisch zwischen dem hydraulischen Motor (13) und dem Instrumentenkopf (5) gekoppelt ist, wobei das Über- oder Untersetzungsgetriebe vorzugsweise in Form eines Spannungswellengetriebes ausgestaltet ist.

**Claims**

1. A minimal invasive medical instrument (1) with
a shank (3) which extends in a longitudinal direction (L),
an instrument head (5) which a movable and/or actuatable and is located at a distal end of the shank (3),
a proximal shank section (9) and a distal shank section (7), wherein the distal shank section (7) can be bent with respect to the proximal shank section (9) about a pivot axis (A) via a joint (11),
at least one fluid channel (15) which leads from the proximal shank section (9) into the distal shank section (7),
wherein the joint (11) comprises a first joint part (17) and a second joint part (19) which is pivotable with respect to the first joint part (17) about the pivot axis (A),
wherein the at least one fluid channel (15) comprises a first channel section (35) in the first joint part (17), a second channel section (45) in the second joint part (19) and a third channel section (51) which extends annularly about the pivot axis (A), **characterised in that**
the third channel section (51) comprises an annular wall section (53) which is formed by the first joint part (17) and a second annular wall section (55) which is formed by the second joint part (19).

2. A minimal-invasive medical instrument (1) according to claim 1, wherein the at least one fluid channel (15) extends at least partly in the direction of the pivot axis (A).

3. A minimal-invasive medical instrument (1) according to claim 1, wherein the joint (11) comprises a pivot body (23) which extends in the direction of the pivot axis (A) and through the annular channel section (51).

4. A minimal-invasive medical instrument (1) according to one of the preceding claims, wherein the first channel section (35) at least in stretches runs radially offset vis-à-vis the second channel section (45) with respect to the longitudinal direction (L).

5. A minimal-invasive medical instrument (1) according to one of the preceding claims, wherein the first annular wall section (53) and the second annular wall section (55) are adjacent to one another in the axial direction of the pivot axis (A).

**6.** A minimal-invasive medical instrument (1) according to one of the preceding claims, wherein the first joint part (17) and the second joint part (19) each comprise contact surfaces (65, 67) which are essentially planar and which in a sealing plane which is orthogonal to the pivot axis (A) sealingly bear on one another around the pivot axis (A).

**7.** A minimal-invasive medical instrument (1) according to one of the preceding claims, wherein the joint (11) comprises an annular sealing element which runs around the pivot axis (A) and which is sealingly arranged between the first joint part (17) and the second joint part (19).

**8.** A minimal-invasive medical instrument (1) according to one of the preceding claims, wherein the first annular wall section (53) and the second annular wall section (55) are adjacent to one another in the radial direction with respect to the pivot axis (A).

**9.** A minimal-invasive medical instrument (1) according to claim 8, wherein the first joint part (17) and the second joint part (19) each comprise contact surfaces (31, 41) which are essentially cylinder-jacket-shaped and which radially bear on one another around the pivot axis (A) in a sealing jacket plane which runs coaxially to the pivot axis (A).

**10.** A minimal-invasive medical instrument (1) according to claim 8 or 9, wherein the joint (11) comprises a pivot body (23) which extends in the direction of the pivot axis (A) and through the annular channel section (51), wherein the pivot body (23) forms a part of the first joint part (17) and is connected to this in a rotationally fixed manner.

**11.** A minimal-invasive medical instrument (1) according to claim 10, wherein a part of the first channel section (35) runs through the pivot body (23).

**12.** A minimal-invasive medical instrument (1) according to one of the claims 10 to 11, wherein the first annular wall section (53) is formed by a, with respect to the pivot axis (A), radial outer surface (41) of the pivot body (23).

**13.** A minimal-invasive medical instrument (1) according to one of the claims 8 to 12, wherein the second annular wall section (55) is formed by a, with regard to the pivot axis (A), radial inner surface (31) of the second joint part (19).

**14.** A minimal-invasive medical instrument (1) according to one of the claims 8 to 13, wherein the pivot axis (A) runs orthogonally to the longitudinal direction (L).

**15.** A minimal-invasive medical instrument (1) according to one of the claims 8 to 13, wherein the pivot axis (A) runs at an angle (a) of 40□ to 60□ to the longitudinal direction (L).

**16.** A minimal-invasive medical instrument (1) according to one of the preceding claims, wherein the instrument head (5) is fluidi-cally drivable and/'or controllable via the at least one fluid channel (15).

**17.** A minimal-invasive medical instrument (1) according to one of the preceding claims, wherein the instrument head (5) comprises at least two degrees of freedom for the movement or actuation and wherein the instrument (1) for the fluidic drive or for the fluidic control of the instrument head (5), for each degree of freedom each comprises at least one separate fluid channel (15a,b,c) which leads from the proximal shank section (9) into the distal shank section (7).

**18.** A minimal-invasive medical instrument (1) according to claim 17, wherein the respective third channel sections (51a,b,c) of the fluid channels (15a, b, c) which run annularly about the pivot axis (A) are arranged parallel to one another, wherein the first annular wall section (53a, b, c) of the fluid channels (15a,b,c) is formed by a, with respect to the pivot axis (A), radial outer surface (41) of the pivot body (23) and the second annular wall section (55a,b,c) of the fluid channels (15a,b, c) is formed by a, with respect to the pivot axis (A), radial inner surface (31) of the second joint part (19).

**19.** A minimal-invasive medical instrument (1) according to one of the preceding claims, wherein the at least one fluid channel (15) at least in sections is formed by way of a generative manufacturing method for one or more parts of the instrument (1).

**20.** A minimal-invasive medical instrument (1) according to one of the preceding claims, wherein the at least one fluid channel (15) fluid-connects a pressurised fluid source which is arranged proximally of the joint (11) to a hydraulic motor (13) which is arranged distally of the joint (11), wherein the hydraulic motor (13) is preferably constructed according to the generator principle, according to the PAD motor principle or the strain wave gear principle.

**21.** A minimal-invasive medical instrument (1) according to claim 20, wherein the at least one fluid channel (15) is fluid-connected to at least one fluid outlet which is arranged distally of the hydraulic motor (13), so that a bio-compatible fluid for the drive of the hydraulic motor (13) can be used as a rinsing fluid or cutting jet fluid

**22.** A minimal-invasive medical instrument (1) according to claim 20 or 21, wherein a step-up gear or a step-down gear is mechanically coupled between the hydraulic motor (13) and the instrument head (5), wherein the step-up or step-down gear is preferably designed in the form of a strain wave gear.

**Revendications**

1. Instrument médical à invasion minimale (1), comportant
une tige (3) s'étendant dans une direction longitudinale (L),
une tête d'instrument mobile et/ou actionnable (5) située à une extrémité distale de la tige (3),
une section de tige proximale (9) et une section de tige distale (7), la section de tige distale (7) pouvant être déployée autour d'un axe de pivotement (A) par rapport à la section de tige proximale (9) par l'intermédiaire d'une articulation (11), et
au moins un canal de fluide (15) menant de la section de tige proximale (9) à la section de tige distale (7),
dans lequel l'articulation (11) présente une première partie d'articulation (17) et une seconde partie d'articulation (19) pouvant pivoter autour de l'axe de pivotement (A) par rapport à la première partie d'articulation (17),
dans lequel le canal de fluide (15), au moins au nombre de un, présente une première section de canal (35) dans la première partie d'articulation (17), une deuxième section de canal (45) dans la seconde partie d'articulation (19), et une troisième section de canal (51) s'étendant de manière annulaire autour de l'axe de pivotement (A),
**caractérisé en ce que** la troisième section de canal (51) présente une première section de paroi annulaire (53) formée par la première partie d'articulation (17) et une seconde section de paroi annulaire (55) formée par la seconde partie d'articulation (19).

2. Instrument médical à invasion minimale (1) selon la revendication 1, dans lequel le canal de fluide (15), au moins au nombre de un, s'étend au moins partiellement dans la direction de l'axe de pivotement (A).

3. Instrument médical à invasion minimale (1) selon la revendication 1 ou 2, dans lequel l'articulation (11) présente un corps d'axe (23), qui s'étend dans la direction de l'axe de pivotement (A) et à travers la section de canal annulaire (51).

4. Instrument médical à invasion minimale (1) selon l'une des revendications précédentes, dans lequel la première section de canal (35) s'étend en étant décalée radialement, au moins par endroits, par rapport à la deuxième section de canal (45) eu égard à la direction longitudinale (L).

5. Instrument médical à invasion minimale (1) selon l'une des revendications précédentes, dans lequel la première section de paroi annulaire (53) et la seconde section de paroi annulaire (55) sont contiguës dans la direction axiale de l'axe de pivotement (A).

6. Instrument médical à invasion minimale (1) selon l'une des revendications précédentes, dans lequel la première partie d'articulation (17) et la seconde partie d'articulation (19) présentent chacune des surfaces de contact sensiblement planes (65, 67) qui, dans un plan d'étanchéité orthogonal à l'axe de pivotement (A), sont contiguës de façon étanche autour de l'axe de pivotement (A).

7. Instrument médical à invasion minimale (1) selon l'une des revendications précédentes, dans lequel l'articulation (11) présente un élément d'étanchéité annulaire qui s'étend autour de l'axe de pivotement (A) et est agencé de manière étanche entre la première partie d'articulation (17) et la seconde partie d'articulation (19).

8. Instrument médical à invasion minimale (1) selon l'une des revendications précédentes, dans lequel la première section de paroi annulaire (53) et la seconde section de paroi annulaire (55) sont contiguës dans la direction radiale eu égard à l'axe de pivotement (A).

9. Instrument médical à invasion minimale (1) selon la revendication 8, dans lequel la première partie d'articulation (17) et la seconde partie d'articulation (19) présentent chacune des surfaces de contact sensiblement en forme de gaine cylindrique (31, 41) qui, dans un plan de gaine d'étanchéité s'étendant coaxialement par rapport à l'axe de pivotement (A), reposent de façon contiguë et de façon étanche autour de l'axe de pivotement (A).

10. Instrument médical à invasion minimale (1) selon la revendication 8 ou 9, dans lequel l'articulation (11) présente un corps d'axe (23) qui s'étend dans la direction de l'axe de pivotement (A) et à travers la section de canal annulaire (51), le corps d'axe (23) formant une partie de la première partie d'articulation (17) et y étant relié de manière solidaire en rotation.

11. Instrument médical à invasion minimale (1) selon la revendication 10, dans lequel une partie de la première section de canal (35) s'étend à travers le corps d'axe (23).

12. Instrument médical à invasion minimale (1) selon l'une des revendications 10 à 11, dans lequel la première section de paroi annulaire (53) est formée par une surface extérieure (41) du corps d'axe (23) qui est radiale par rapport à l'axe de pivotement (A).

**13.** Instrument médical à invasion minimale (1) selon l'une des revendications 8 à 12, dans lequel la seconde section de paroi annulaire (55) est formée par une surface intérieure (31) de la seconde partie d'articulation (19) qui est radiale par rapport à l'axe de pivotement (A).

**14.** Instrument médical à invasion minimale (1) selon l'une des revendications 8 à 13, dans lequel l'axe de pivotement (A) est orthogonal à la direction longitudinale (L).

**15.** Instrument médical à invasion minimale (1) selon l'une des revendications 8 à 13, dans lequel l'axe de pivotement (A) s'étend en formant un angle (a) de 40° à 60° par rapport à la direction longitudinale (L).

**16.** Instrument médical à invasion minimale (1) selon l'une des revendications précédentes, dans lequel la tête d'instrument (5) peut être entraînée et/ou commandée de manière fluidique via le canal de fluide (15), au moins au nombre de un.

**17.** Instrument médical à invasion minimale (1) selon l'une des revendications précédentes, dans lequel la tête d'instrument (5) présente au moins deux degrés de liberté de déplacement ou d'actionnement, et dans lequel l'instrument (1), pour l'entraînement ou pour la commande fluidique de la tête d'instrument (5), présente pour chaque degré de liberté respectif au moins un canal de fluide séparé (15a, b, c) menant de la section de tige proximale (9) à la section de tige distale (7).

**18.** Instrument médical à invasion minimale (1) selon la revendication 17, dans lequel les troisièmes sections de canal respectives (51a, b, c) des canaux de fluide (15a, b, c) s'étendant de manière annulaire autour de l'axe de pivotement (A) sont agencées parallèlement les unes aux autres, dans lequel la première section de paroi annulaire (53a, b, c) respective des canaux de fluide (15a, b, c) est formée par une surface extérieure (41) du corps d'axe (23) qui est radiale par rapport à l'axe de pivotement (A), et la deuxième section de paroi annulaire (55a, b, c) respective des canaux de fluide (15a, b, c) est formée par une surface intérieure (31) de la seconde partie d'articulation (19) qui est radiale par rapport à l'axe de pivotement (A).

**19.** Instrument médical à invasion minimale (1) selon l'une des revendications précédentes, dans lequel le canal de fluide (15), au moins au nombre de un, est exécuté au moins par endroits au moyen d'un procédé de fabrication générative pour une ou plusieurs parties de l'instrument (1).

**20.** Instrument médical à invasion minimale (1) selon l'une des revendications précédentes, dans lequel le canal de fluide (15), au moins au nombre de un, relie de manière fluidique une source de pression de fluide agencée de manière proximale par rapport à l'articulation (11) à un moteur hydraulique (13) agencé de manière distale par rapport à l'articulation (11), dans lequel le moteur hydraulique (13) est de préférence construit selon le principe du générateur, le principe du moteur Kappel ou le principe de la transmission à démultiplicateur harmonique.

**21.** Instrument médical à invasion minimale (1) selon la revendication 20, dans lequel le canal de fluide (15), au moins au nombre de un, est relié de manière fluidique à au moins une sortie de fluide agencée de manière distale par rapport au moteur hydraulique (13), de sorte qu'un fluide biocompatible pour entraîner le moteur hydraulique (13) peut être utilisé en tant que fluide de rinçage ou fluide pour jet de coupe.

**22.** Instrument médical à invasion minimale (1) selon la revendication 20 ou 21, dans lequel un multiplicateur ou un démultiplicateur est couplé mécaniquement entre le moteur hydraulique (13) et la tête d'instrument (5), le multiplicateur ou le démultiplicateur étant de préférence conçu sous la forme d'une transmission à démultiplicateur harmonique.

Fig. 1

EP 3 498 198 B1

Fig. 2

Fig. 3

Fig. 4

Fig. 5

Fig. 6

Fig.7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4319345 C1
- US 20060235368 A1
- DE 19742669 A1
- US 7963963 B2
- US 2007095264 A1